# EUROPÄISCHE PATENTANMELDUNG

(11) **EP 1 316 609 A1**
(43) Veröffentlichungstag der Anmeldung: **04.06.2003**
(21) Anmeldenummer: 02025667.3
(22) Anmeldetag: 20.11.2002
(51) Int. Cl.: C12N 15/54, C12N 5/10, C12N 9/10, C12Q 1/02, C12Q 1/48, C12N 1/15, C07K 16/14, C12N 15/82, G01N 33/50

(54) **Acetoacetyl-CoA Thiolase und deren Verwendung zum Identifizieren von neuen fungizid wirksamen Substanzen**

(30) Priorität: 03.12.2001 DE 10159264
(71) Anmelder: Bayer CropScience AG, 40789 Monheim (DE)
(72) Erfinder: Schreier, Peter, Dr., 50674 Köln (DE); Ebbert, Ronald, Dr., 40789 Köln (DE); Oehmen, Edith, 42799 Leichlingen (DE)

(57) **Zusammenfassung**

Die Erfindung betrifft Nukleinsäuren, die für Polypeptide aus Pilzen mit der biologischen Aktivität von Acetoacetyl-CoA-Thiolase kodieren, die davon kodierten Polypeptide sowie deren Verwendung als Targets für Fungizide und deren Verwendung zum Identifizieren von neuen, fungizid wirksamen Verbindungen sowie Verfahren zum Auffinden von Modulatoren dieser Polypeptide und schließlich transgene Organismen enthaltend für Polypeptide aus Pilzen mit der Funktion einer Acetoacetyl-CoA-Thiolase kodierende Sequenzen.

SEQUENCE LISTING

<110>  Bayer AG

<120>  Verwendung von Acetoacetyl-CoA-Thiolase zum Identifizieren von
neuen fungizid wirksamen Substanzen

<130>  Le A 35 574

<160>  15

<170>  PatentIn version 3.1

<210>  1

<211>  1911

<212>  DNA

<213>  Ustilago maydis

<400>  1
gttctcacgc ttgataatga tagcagaagc gccaccacca ccgttgcaga caccagcgca      60

gccgtattgg ccgggcttga gcgcgtgggc cagcgtaaca acaatcctag cacctgaaga     120

accgataggg tgaccgagtg aaacaccacc accgagaacg ttgaccttgc tagcgtcgag     180

gccgagcatc tggttgttag ccaacgcgac agccgagaaa gcctcgttga tttcgaacag     240

agcaatgtcg tcctttgtca aaccggctcg ctcgagcgcc ttgggaatgg cataagcggg     300

cgcaatgggg aagtcgatcg gtgcacatgc tgcatcggca aacgctacaa tcttggccaa     360

aggcttaatg cccaacttgg ccacctcggc ctcggaagct agcacaacgg ccgaagcacc     420

gtcgttgagc gtcgacgcat tggcagcagt gatggttccg ttcttgtcga acaccggtcg     480

aagcgatggg atcttttcga gcttgacgtt cttgtactcc tcgtcttcgc tgatcacgac     540

atcgcccttc ttgtccgaga tggtgacagg agcgatctcg ttagcgaatg cattggcctt     600

ccatgcctcg gcggagcgac ggtacgattc aatcgcaaag gcgtcctgct gctcacgcgt     660

aattgagagc ttcttggcag tgttctcggc acagttgccc atggccacct ggttgtagac     720

atcgtgcaga ccgtccttga cgatcgcatc ggtcgcctgg acatggccgt aggtgttgcc     780

tcggggaaga tagtagctgt tcgaaacaaa cgaaacaaaa gaaacaaaaa gggaaaaatg     840

tcagacaaga gcttcctgag acagactcaa ctgccgccgt tggggtgttc actcccctgt 900

agctctcttt cgaaaggcaa tgtcgcgacg ccaacagcct gtgcgaatgc aactatgggc 960

ctcctcatat gtgctagttt tcgcttgtga tccttgatcc caagctgtag cctggcgtcg 1020

actggcgccg cgcatgacac ccgagtcgag agtctcgggc caggcgggca aaccaacgtc 1080

cgaacgacat tttccccaca tgtactggaa tgttaggccg acaccacgaa ttcgatccga 1140

acggcaaata aaaatgcggg tgtaccggtc cagcttttgc ccttgccaag agttttgctt 1200

gggttgagcc gtctaggcca gagcaccaaa caaacgcttc gaagtagcaa cctcagcgca 1260

cgctccccct cttctgcagt agagacatgc tttctctaaa gctgatgcaa tgagagcctg 1320

gataacgata cttttgtcgg tacttacggg gcgttagaca tagactccat gccaccggcg 1380

accatgatac tgcgctggcc gagagcaatg ttctgggcgg cgagcgagat ggccttcata 1440

ccggaagcgc acaccttgtt aatggtggta gcctcggtgg tgtcggggca gccagccttg 1500

agcgcaacct gacgggcggg agcctgaccg acgttgcctt ggagcacatt gcccatgtag 1560

acctcctcga tctggtcggg cttgagaccg gcacgttgga tggctgcctt tacggccacc 1620

acaccgagct caggagcggt ggcctttttg agcacaccgt tgaacgagcc gatgggcgtt 1680

cgggcggcgg aaacgatgaa gacgtcgttg atttgtgact aatgtgtcca tcgttgatgc 1740

cgtttgtaga gcgtagagga gaggggtggt atcgagaagt cagtcaatct ggatgttgcg 1800

ggcaccggag tacgtgtgtg taggtgcaac tttgcgactg caatgcagac ttaccataat 1860

tctagctgtc tgagaaagtc gtcggacggc ggtttggtag aaagctggca t 1911

<210> 2

<211> 1914

<212> DNA

<213> Ustilago maydis

<400> 2
atgccagctt tctaccaaac cgccgtccga cgactttctc agacagctag aattatggta 60

agtctgcatt gcagtcgcaa agttgcacct acacacacgt actccggtgc ccgcaacatc 120

cagattgact gacttctcga taccacccct ctcctctacg ctctacaaac ggcatcaacg 180

atggacacat tagtcacaaa tcaacgacgt cttcatcgtt tccgccgccc gaacgcccat 240

cggctcgttc aacggtgtgc tcaaaaaggc caccgctcct gagctcggtg tggtggccgt 300

aaaggcagcc atccaacgtg ccggtctcaa gcccgaccag atcgaggagg tctacatggg 360

caatgtgctc caaggcaacg tcggtcaggc tcccgcccgt caggttgcgc tcaaggctgg 420

ctgccccgac accaccgagg ctaccaccat taacaaggtg tgcgcttccg gtatgaaggc 480

catctcgctc gccgcccaga acattgctct cggccagcgc agtatcatgg tcgccggtgg 540

catggagtct atgtctaacg ccccgtaagt accgacaaaa gtatcgttat ccaggctctc 600

attgcatcag ctttagagaa agcatgtctc tactgcagaa gagggggagc gtgcgctgag 660

gttgctactt cgaagcgttt gtttggtgct ctggcctaga cggctcaacc caagcaaaac 720

tcttggcaag ggcaaaagct ggaccggtac acccgcattt ttatttgccg ttcggatcga 780

attcgtggtg tcggcctaac attccagtac atgtggggaa aatgtcgttc ggacgttggt 840

ttgcccgcct ggcccgagac tctcgactcg ggtgtcatgc gcggcgccag tcgacgccag 900

gctacagctt gggatcaagg atcacaagcg aaaactagca catatgagga ggcccatagt 960

tgcattcgca caggctgttg gcgtcgcgac attgcctttc gaaagagagc tacaggggag 1020

tgaacacccc aacggcggca gttgagtctg tctcaggaag ctcttgtctg acatttttcc 1080

ctttttgttt cttttgtttc gtttgtttcg aacagctact atcttccccg aggcaacacc 1140

tacggccatg tccaggcgac cgatgcgatc gtcaaggacg gtctgcacga tgtctacaac 1200

caggtggcca tgggcaactg tgccgagaac actgccaaga agctctcaat tacgcgtgag 1260

cagcaggacg cctttgcgat tgaatcgtac cgtcgctccg ccgaggcatg gaaggccaat 1320

gcattcgcta acgagatcgc tcctgtcacc atctcggaca agaagggcga tgtcgtgatc 1380

agcgaagacg aggagtacaa gaacgtcaag ctcgaaaaga tcccatcgct tcgaccggtg 1440

ttcgacaaga acggaaccat cactgctgcc aatgcgtcga cgctcaacga cggtgcttcg 1500

gccgttgtgc tagcttccga ggccgaggtg gccaagttgg gcattaagcc tttggccaag 1560

attgtagcgt ttgccgatgc agcatgtgca ccgatcgact tccccattgc gcccgcttat 1620

gccattccca aggcgctcga gcgagccggt ttgacaaagg acgacattgc tctgttcgaa 1680

atcaacgagg ctttctcggc tgtcgcgttg gctaacaacc agatgctcgg cctcgacgct 1740

agcaaggtca acgttctcgg tggtggtgtt tcactcggtc accctatcgg ttcttcaggt 1800

gctaggattg ttgttacgct ggcccacgcg ctcaagcccg gccaatacgg ctgcgctggt 1860

gtctgcaacg gtggtggtgg cgcttctgct atcattatca agcgtgagaa ctaa 1914

<210> 3

<211> 1227

<212> DNA

<213> Ustilago maydis

<220>

<221> CDS

<222>  (1)..(1224)

<223>

<400>  3
atg cca gct ttc tac caa acc gcc gtc cga cga ctt tct cag aca gct       48
Met Pro Ala Phe Tyr Gln Thr Ala Val Arg Arg Leu Ser Gln Thr Ala
1               5                   10                  15

aga att atg tca caa atc aac gac gtc ttc atc gtt tcc gcc gcc cga       96
Arg Ile Met Ser Gln Ile Asn Asp Val Phe Ile Val Ser Ala Ala Arg
            20                  25                  30

acg ccc atc ggc tcg ttc aac ggt gtg ctc aaa aag gcc acc gct cct      144
Thr Pro Ile Gly Ser Phe Asn Gly Val Leu Lys Lys Ala Thr Ala Pro
        35                  40                  45

gag ctc ggt gtg gtg gcc gta aag gca gcc atc caa cgt gcc ggt ctc      192
Glu Leu Gly Val Val Ala Val Lys Ala Ala Ile Gln Arg Ala Gly Leu
    50                  55                  60

aag ccc gac cag atc gag gag gtc tac atg ggc aat gtg ctc caa ggc      240
Lys Pro Asp Gln Ile Glu Glu Val Tyr Met Gly Asn Val Leu Gln Gly
65                  70                  75                  80

aac gtc ggt cag gct ccc gcc cgt cag gtt gcg ctc aag gct ggc tgc      288
Asn Val Gly Gln Ala Pro Ala Arg Gln Val Ala Leu Lys Ala Gly Cys
                85                  90                  95

ccc gac acc acc gag gct acc acc att aac aag gtg tgc gct tcc ggt      336
Pro Asp Thr Thr Glu Ala Thr Thr Ile Asn Lys Val Cys Ala Ser Gly
            100                 105                 110

atg aag gcc atc tcg ctc gcc gcc cag aac att gct ctc ggc cag cgc      384
Met Lys Ala Ile Ser Leu Ala Ala Gln Asn Ile Ala Leu Gly Gln Arg
        115                 120                 125

agt atc atg gtc gcc ggt ggc atg gag tct atg tct aac gcc ccc tac      432
Ser Ile Met Val Ala Gly Gly Met Glu Ser Met Ser Asn Ala Pro Tyr
    130                 135                 140

tat ctt ccc cga ggc aac acc tac ggc cat gtc cag gcg acc gat gcg      480
Tyr Leu Pro Arg Gly Asn Thr Tyr Gly His Val Gln Ala Thr Asp Ala
145                 150                 155                 160

atc gtc aag gac ggt ctg cac gat gtc tac aac cag gtg gcc atg ggc      528
Ile Val Lys Asp Gly Leu His Asp Val Tyr Asn Gln Val Ala Met Gly
                165                 170                 175

aac tgt gcc gag aac act gcc aag aag ctc tca att acg cgt gag cag      576
Asn Cys Ala Glu Asn Thr Ala Lys Lys Leu Ser Ile Thr Arg Glu Gln
            180                 185                 190

cag gac gcc ttt gcg att gaa tcg tac cgt cgc tcc gcc gag gca tgg      624
Gln Asp Ala Phe Ala Ile Glu Ser Tyr Arg Arg Ser Ala Glu Ala Trp
        195                 200                 205

aag gcc aat gca ttc gct aac gag atc gct cct gtc acc atc tcg gac      672
Lys Ala Asn Ala Phe Ala Asn Glu Ile Ala Pro Val Thr Ile Ser Asp
    210                 215                 220

aag aag ggc gat gtc gtg atc agc gaa gac gag gag tac aag aac gtc      720

Lys Lys Gly Asp Val Val Ile Ser Glu Asp Glu Glu Tyr Lys Asn Val
225                 230                 235                 240

aag ctc gaa aag atc cca tcg ctt cga ccg gtg ttc gac aag aac gga      768
Lys Leu Glu Lys Ile Pro Ser Leu Arg Pro Val Phe Asp Lys Asn Gly
                245                 250                 255

acc atc act gct gcc aat gcg tcg acg ctc aac gac ggt gct tcg gcc      816
Thr Ile Thr Ala Ala Asn Ala Ser Thr Leu Asn Asp Gly Ala Ser Ala
            260                 265                 270

gtt gtg cta gct tcc gag gcc gag gtg gcc aag ttg ggc att aag cct      864
Val Val Leu Ala Ser Glu Ala Glu Val Ala Lys Leu Gly Ile Lys Pro
        275                 280                 285

ttg gcc aag att gta gcg ttt gcc gat gca gca tgt gca ccg atc gac      912
Leu Ala Lys Ile Val Ala Phe Ala Asp Ala Ala Cys Ala Pro Ile Asp
    290                 295                 300

ttc ccc att gcg ccc gct tat gcc att ccc aag gcg ctc gag cga gcc      960
Phe Pro Ile Ala Pro Ala Tyr Ala Ile Pro Lys Ala Leu Glu Arg Ala
305                 310                 315                 320

ggt ttg aca aag gac gac att gct ctg ttc gaa atc aac gag gct ttc      1008
Gly Leu Thr Lys Asp Asp Ile Ala Leu Phe Glu Ile Asn Glu Ala Phe
                325                 330                 335

tcg gct gtc gcg ttg gct aac aac cag atg ctc ggc ctc gac gct agc      1056
Ser Ala Val Ala Leu Ala Asn Asn Gln Met Leu Gly Leu Asp Ala Ser
            340                 345                 350

aag gtc aac gtt ctc ggt ggt ggt gtt tca ctc ggt cac cct atc ggt      1104
Lys Val Asn Val Leu Gly Gly Gly Val Ser Leu Gly His Pro Ile Gly
        355                 360                 365

tct tca ggt gct agg att gtt gtt acg ctg gcc cac gcg ctc aag ccc      1152
Ser Ser Gly Ala Arg Ile Val Val Thr Leu Ala His Ala Leu Lys Pro
    370                 375                 380

ggc caa tac ggc tgc gct ggt gtc tgc aac ggt ggt ggt ggc gct tct      1200
Gly Gln Tyr Gly Cys Ala Gly Val Cys Asn Gly Gly Gly Gly Ala Ser
385                 390                 395                 400

gct atc att atc aag cgt gag aac taa                                  1227
Ala Ile Ile Ile Lys Arg Glu Asn
                405

<210>  4

<211>  408

<212>  PRT

<213>  Ustilago maydis

<400>  4

Met Pro Ala Phe Tyr Gln Thr Ala Val Arg Arg Leu Ser Gln Thr Ala
1               5                   10                  15

Arg Ile Met Ser Gln Ile Asn Asp Val Phe Ile Val Ser Ala Ala Arg
            20                  25                  30

Thr Pro Ile Gly Ser Phe Asn Gly Val Leu Lys Lys Ala Thr Ala Pro
        35                  40                  45

Glu Leu Gly Val Val Ala Val Lys Ala Ala Ile Gln Arg Ala Gly Leu
    50                  55                  60

Lys Pro Asp Gln Ile Glu Glu Val Tyr Met Gly Asn Val Leu Gln Gly
65                  70                  75                  80

Asn Val Gly Gln Ala Pro Ala Arg Gln Val Ala Leu Lys Ala Gly Cys
                85                  90                  95

Pro Asp Thr Thr Glu Ala Thr Thr Ile Asn Lys Val Cys Ala Ser Gly
            100                 105                 110

Met Lys Ala Ile Ser Leu Ala Ala Gln Asn Ile Ala Leu Gly Gln Arg
        115                 120                 125

Ser Ile Met Val Ala Gly Gly Met Glu Ser Met Ser Asn Ala Pro Tyr
    130                 135                 140

Tyr Leu Pro Arg Gly Asn Thr Tyr Gly His Val Gln Ala Thr Asp Ala
145                 150                 155                 160

Ile Val Lys Asp Gly Leu His Asp Val Tyr Asn Gln Val Ala Met Gly
                165                 170                 175

Asn Cys Ala Glu Asn Thr Ala Lys Lys Leu Ser Ile Thr Arg Glu Gln
            180                 185                 190

Gln Asp Ala Phe Ala Ile Glu Ser Tyr Arg Arg Ser Ala Glu Ala Trp
        195                 200                 205

Lys Ala Asn Ala Phe Ala Asn Glu Ile Ala Pro Val Thr Ile Ser Asp
    210                 215                 220

Lys Lys Gly Asp Val Val Ile Ser Glu Asp Glu Glu Tyr Lys Asn Val
225                 230                 235                 240

Lys Leu Glu Lys Ile Pro Ser Leu Arg Pro Val Phe Asp Lys Asn Gly
                245                 250                 255

Thr Ile Thr Ala Ala Asn Ala Ser Thr Leu Asn Asp Gly Ala Ser Ala
            260                 265                 270

Val Val Leu Ala Ser Glu Ala Glu Val Ala Lys Leu Gly Ile Lys Pro
      275                 280                 285

Leu Ala Lys Ile Val Ala Phe Ala Asp Ala Ala Cys Ala Pro Ile Asp
    290                 295                 300

Phe Pro Ile Ala Pro Ala Tyr Ala Ile Pro Lys Ala Leu Glu Arg Ala
305                 310                 315                 320

Gly Leu Thr Lys Asp Asp Ile Ala Leu Phe Glu Ile Asn Glu Ala Phe
                325                 330                 335

Ser Ala Val Ala Leu Ala Asn Asn Gln Met Leu Gly Leu Asp Ala Ser
            340                 345                 350

Lys Val Asn Val Leu Gly Gly Gly Val Ser Leu Gly His Pro Ile Gly
      355                 360                 365

Ser Ser Gly Ala Arg Ile Val Val Thr Leu Ala His Ala Leu Lys Pro
    370                 375                 380

Gly Gln Tyr Gly Cys Ala Gly Val Cys Asn Gly Gly Gly Gly Ala Ser
385                 390                 395                 400

Ala Ile Ile Ile Lys Arg Glu Asn
                405

<210>  5

<211>  1197

<212>  DNA

<213>  Saccharomyces cerevisiae

<220>

<221>  CDS

<222>  (1)..(1194)

<223>

<400>  5
atg tct cag aac gtt tac att gta tcg act gcc aga acc cca att ggt       48
Met Ser Gln Asn Val Tyr Ile Val Ser Thr Ala Arg Thr Pro Ile Gly
1               5                   10                  15

tca ttc cag ggt tct cta tcc tcc aag aca gca gtg gaa ttg ggt gct       96
Ser Phe Gln Gly Ser Leu Ser Ser Lys Thr Ala Val Glu Leu Gly Ala
            20                  25                  30

gtt gct tta aaa ggc gcc ttg gct aag gtt cca gaa ttg gat gca tcc 144
Val Ala Leu Lys Gly Ala Leu Ala Lys Val Pro Glu Leu Asp Ala Ser
        35                  40                  45

aag gat ttt gac gaa att att ttt ggt aac gtt ctt tct gcc aat ttg 192
Lys Asp Phe Asp Glu Ile Ile Phe Gly Asn Val Leu Ser Ala Asn Leu
    50                  55                  60

ggc caa gct ccg gcc aga caa gtt gct ttg gct gcc ggt ttg agt aat 240
Gly Gln Ala Pro Ala Arg Gln Val Ala Leu Ala Ala Gly Leu Ser Asn
65                  70                  75                  80

cat atc gtt gca agc aca gtt aac aag gtc tgt gca tcc gct atg aag 288
His Ile Val Ala Ser Thr Val Asn Lys Val Cys Ala Ser Ala Met Lys
                85                  90                  95

gca atc att ttg ggt gct caa tcc atc aaa tgt ggt aat gct gat gtt 336
Ala Ile Ile Leu Gly Ala Gln Ser Ile Lys Cys Gly Asn Ala Asp Val
            100                 105                 110

gtc gta gct ggt ggt tgt gaa tct atg act aac gca cca tac tac atg 384
Val Val Ala Gly Gly Cys Glu Ser Met Thr Asn Ala Pro Tyr Tyr Met
        115                 120                 125

cca gca gcc cgt gcg ggt gcc aaa ttt ggc caa act gtt ctt gtt gat 432
Pro Ala Ala Arg Ala Gly Ala Lys Phe Gly Gln Thr Val Leu Val Asp
    130                 135                 140

ggt gtc gaa aga gat ggg ttg aac gat gcg tac gat ggt cta gcc atg 480
Gly Val Glu Arg Asp Gly Leu Asn Asp Ala Tyr Asp Gly Leu Ala Met
145                 150                 155                 160

ggt gta cac gca gaa aag tgt gcc cgt gat tgg gat att act aga gaa 528
Gly Val His Ala Glu Lys Cys Ala Arg Asp Trp Asp Ile Thr Arg Glu
                165                 170                 175

caa caa gac aat ttt gcc atc gaa tcc tac caa aaa tct caa aaa tct 576
Gln Gln Asp Asn Phe Ala Ile Glu Ser Tyr Gln Lys Ser Gln Lys Ser
            180                 185                 190

caa aag gaa ggt aaa ttc gac aat gaa att gta cct gtt acc att aag 624
Gln Lys Glu Gly Lys Phe Asp Asn Glu Ile Val Pro Val Thr Ile Lys
        195                 200                 205

gga ttt aga ggt aag cct gat act caa gtc acg aag gac gag gaa cct 672
Gly Phe Arg Gly Lys Pro Asp Thr Gln Val Thr Lys Asp Glu Glu Pro
    210                 215                 220

gct aga tta cac gtt gaa aaa ttg aga tct gca agg act gtt ttc caa 720
Ala Arg Leu His Val Glu Lys Leu Arg Ser Ala Arg Thr Val Phe Gln
225                 230                 235                 240

aaa gaa aac ggt act gtt act gcc gct aac gct tct cca atc aac gat 768
Lys Glu Asn Gly Thr Val Thr Ala Ala Asn Ala Ser Pro Ile Asn Asp
                245                 250                 255

ggt gct gca gcc gtc atc ttg gtt tcc gaa aaa gtt ttg aag gaa aag 816
Gly Ala Ala Ala Val Ile Leu Val Ser Glu Lys Val Leu Lys Glu Lys
            260                 265                 270

aat ttg aag cct ttg gct att atc aaa ggt tgg ggt gag gcc gct cat 864
Asn Leu Lys Pro Leu Ala Ile Ile Lys Gly Trp Gly Glu Ala Ala His
        275                 280                 285

caa cca gct gat ttt aca tgg gct cca tct ctt gca gtt cca aag gct     912
Gln Pro Ala Asp Phe Thr Trp Ala Pro Ser Leu Ala Val Pro Lys Ala
    290                 295                 300

ttg aaa cat gct ggc atc gaa gac atc aat tct gtt gat tac ttt gaa     960
Leu Lys His Ala Gly Ile Glu Asp Ile Asn Ser Val Asp Tyr Phe Glu
305                 310                 315                 320

ttc aat gaa gcc ttt tcg gtt gtc ggt ttg gtg aac act aag att ttg     1008
Phe Asn Glu Ala Phe Ser Val Val Gly Leu Val Asn Thr Lys Ile Leu
                325                 330                 335

aag cta gac cca tct aag gtt aat gta tat ggt ggt gct gtt gct cta     1056
Lys Leu Asp Pro Ser Lys Val Asn Val Tyr Gly Gly Ala Val Ala Leu
            340                 345                 350

ggt cac cca ttg ggt tgt tct ggt gct aga gtg gtt gtt aca ctg cta     1104
Gly His Pro Leu Gly Cys Ser Gly Ala Arg Val Val Val Thr Leu Leu
        355                 360                 365

tcc atc tta cag caa gaa gga ggt aag atc ggt gtt gcc gcc att tgt     1152
Ser Ile Leu Gln Gln Glu Gly Gly Lys Ile Gly Val Ala Ala Ile Cys
    370                 375                 380

aat ggt ggt ggt ggt gct tcc tct att gtc att gaa aag ata tga         1197
Asn Gly Gly Gly Gly Ala Ser Ser Ile Val Ile Glu Lys Ile
385                 390                 395

<210>  6

<211>  398

<212>  PRT

<213>  Saccharomyces cerevisiae

<400>  6

Met Ser Gln Asn Val Tyr Ile Val Ser Thr Ala Arg Thr Pro Ile Gly
1               5                   10                  15

Ser Phe Gln Gly Ser Leu Ser Ser Lys Thr Ala Val Glu Leu Gly Ala
            20                  25                  30

Val Ala Leu Lys Gly Ala Leu Ala Lys Val Pro Glu Leu Asp Ala Ser
        35                  40                  45

Lys Asp Phe Asp Glu Ile Ile Phe Gly Asn Val Leu Ser Ala Asn Leu
    50                  55                  60

Gly Gln Ala Pro Ala Arg Gln Val Ala Leu Ala Ala Gly Leu Ser Asn
65                  70                  75                  80

His Ile Val Ala Ser Thr Val Asn Lys Val Cys Ala Ser Ala Met Lys
                85                  90                  95

Ala Ile Ile Leu Gly Ala Gln Ser Ile Lys Cys Gly Asn Ala Asp Val
            100                 105                 110

Val Val Ala Gly Gly Cys Glu Ser Met Thr Asn Ala Pro Tyr Tyr Met
        115                 120                 125

Pro Ala Ala Arg Ala Gly Ala Lys Phe Gly Gln Thr Val Leu Val Asp
    130                 135                 140

Gly Val Glu Arg Asp Gly Leu Asn Asp Ala Tyr Asp Gly Leu Ala Met
145                 150                 155                 160

Gly Val His Ala Glu Lys Cys Ala Arg Asp Trp Asp Ile Thr Arg Glu
                165                 170                 175

Gln Gln Asp Asn Phe Ala Ile Glu Ser Tyr Gln Lys Ser Gln Lys Ser
            180                 185                 190

Gln Lys Glu Gly Lys Phe Asp Asn Glu Ile Val Pro Val Thr Ile Lys
        195                 200                 205

Gly Phe Arg Gly Lys Pro Asp Thr Gln Val Thr Lys Asp Glu Glu Pro
    210                 215                 220

Ala Arg Leu His Val Glu Lys Leu Arg Ser Ala Arg Thr Val Phe Gln
225                 230                 235                 240

Lys Glu Asn Gly Thr Val Thr Ala Ala Asn Ala Ser Pro Ile Asn Asp
                245                 250                 255

Gly Ala Ala Ala Val Ile Leu Val Ser Glu Lys Val Leu Lys Glu Lys
            260                 265                 270

Asn Leu Lys Pro Leu Ala Ile Ile Lys Gly Trp Gly Glu Ala Ala His
        275                 280                 285

Gln Pro Ala Asp Phe Thr Trp Ala Pro Ser Leu Ala Val Pro Lys Ala
    290                 295                 300

Leu Lys His Ala Gly Ile Glu Asp Ile Asn Ser Val Asp Tyr Phe Glu
305                 310                 315                 320

Phe Asn Glu Ala Phe Ser Val Val Gly Leu Val Asn Thr Lys Ile Leu
                325                 330                 335

Lys Leu Asp Pro Ser Lys Val Asn Val Tyr Gly Gly Ala Val Ala Leu
            340                 345                 350

Gly His Pro Leu Gly Cys Ser Gly Ala Arg Val Val Val Thr Leu Leu
        355                 360                 365

Ser Ile Leu Gln Gln Glu Gly Gly Lys Ile Gly Val Ala Ala Ile Cys
    370                 375                 380

Asn Gly Gly Gly Gly Ala Ser Ser Ile Val Ile Glu Lys Ile
385                 390                 395

<210>  7

<211>  25

<212>  DNA

<213>  Primer hph-Nco/Bam

<400>  7
gcggatccca tggaaaagcc tgaac                                          25

<210>  8

<211>  32

<212>  DNA

<213>  Primer hph-stop

<400>  8
gggcggccgc tctattcctt tgccctcgga cg                                  32

<210>  9

<211>  44

<212>  DNA

<213>  Primer 3-hph

<400>  9
cggagctcgg ccactcaggc ctcatgtttg acagcttatc atcg                     44

<210>  10

<211>  45

<212>  DNA

<213>  Primer 5-hsp

<400> 10
cgctcgaggc ctagatggcc gaacgtggta actaccagcg agttc 45

<210> 11

<211> 35

<212> DNA

<213> Primer hsp-Nco

<400> 11
cggaagcttc catggccccc cgggatctgg caacg 35

<210> 12

<211> 23

<212> DNA

<213> Primer lacc11

<400> 12
gaagattcgt gattgaggaa tcc 23

<210> 13

<211> 43

<212> DNA

<213> Primer lacc12

<400> 13
gtcggcctga gtggccgttg atttgtgact aatgtgtcca tcg 43

<210> 14

<211> 41

<212> DNA

<213> Primer racc11

<400> 14
gacggccatc taggccacta ataagctgct cgcttcatga c 41

<210> 15

<211>  23

<212>  DNA

<213>  Primer racc12

<400>  15
gaccgtcatg gatcgaacca tgg                                              23

## Beschreibung

Die Erfindung betrifft Nukleinsäuren, die für Polypeptide aus Pilzen mit der biologischen Aktivität von Acetoacetyl-CoA-Thiolase kodieren, die davon kodierten Polypeptide sowie deren Verwendung als Targets für Fungizide und deren Verwendung zum Identifizieren von neuen, fungizid wirksamen Verbindungen sowie Verfahren zum Auffinden von Modulatoren dieser Polypeptide und schließlich transgene Organismen enthaltend für Polypeptide aus Pilzen mit der Funktion einer Acetoacetyl-CoA-Thiolase kodierende Sequenzen.

Unerwünschtes Pilzwachstum, dass z.B. in der Landwirtschaft jedes Jahr zu beträchtlichen Schäden führt, kann durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von so genannten Hochdurchsatz- bzw. Ultrahochdurchsatzverfahren [(U)HTS = (ultra) high throughput screening]. Dabei werden in einem automatisierten Verfahren eine große Anzahl von Einzelsubstanzen hinsichtlich ihrer Wirkung auf Einzelzellen, individuelle Genprodukte oder Gene getestet. Wird für bestimmte Substanzen eine Wirkung nachgewiesen, so können diese in herkömmlichen Screening-Verfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben. Ein Beispiel für ein solches Fungizid ist z.B. der Wirkstoff Carpropamid, der die Melaninbiosynthese des Pilzes hemmt und so die Ausprägung intakter Appressorien (Haftorgane) verhindert (Kurahashi et al., 1997). Es gibt jedoch nur sehr wenige bekannte Genprodukte, die für Pilze eine solche Rolle spielen. Darüber hinaus sind Fungizide bekannt, die durch die Hemmung entsprechender Biosynthesewege zur Auxotrophie der Zielzellen und in der Folge zum Verlust der Pathogenität führen. So führt z.B. die Inhibition der Adenosin Deaminase durch Ethirimol in *Blumeria graminis* zu einer signifikanten Inhibition.

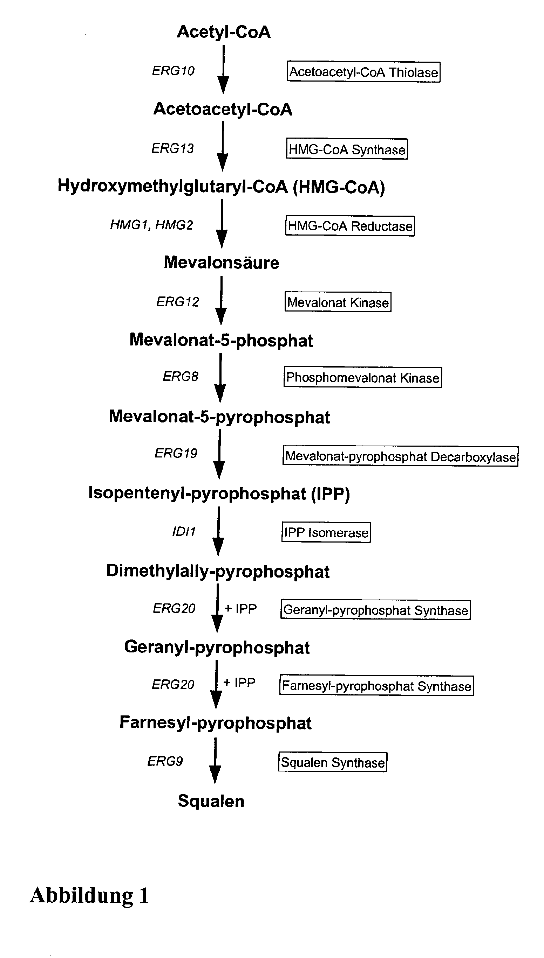

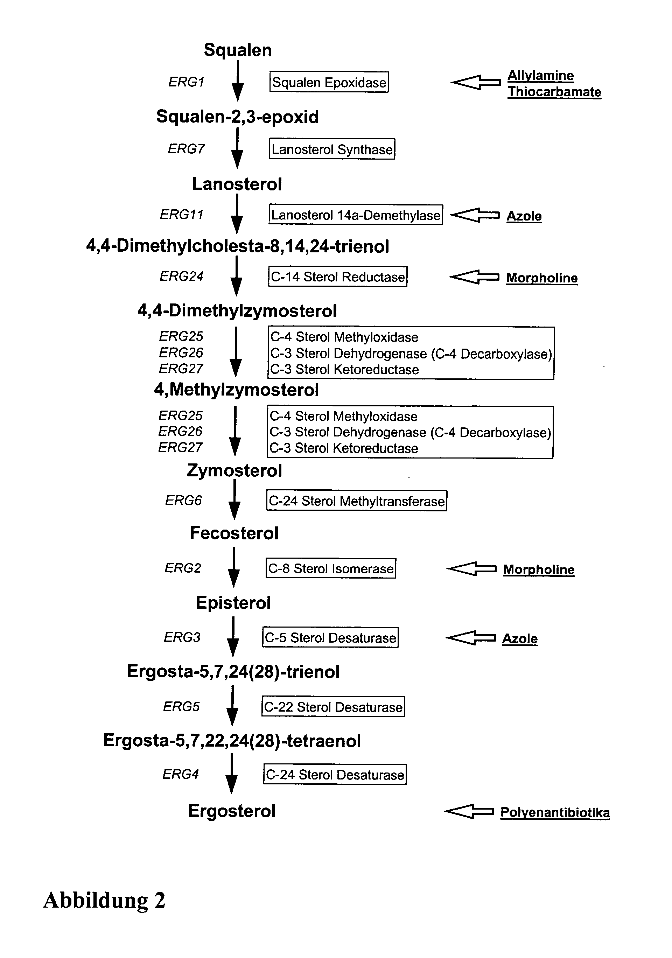

Der Mevalonat-Pathway (vgl. Abb. 1 und Abb. 2) ist ein essentieller Stoffwechselweg, denn seine Produkte schließen alle Isopren-haltigen Verbindungen wie Sterole, Chinone, Dolichol und isopentenyliertes Adenosin in tRNAs ein. Außerdem werden einige Proteine post-translational durch die Isoprenoidgruppen Farnesyl und Geranylgeranyl modifiziert. Mevalonat, der Precursor aller Isoprenoide, wird in drei Schritten gebildet (siehe Abb. 1).

In Eukaryoten und Prokaryoten existieren zwei Klassen von Thiolasen, die sich durch die Kettenlänge ihrer Substrate für die Thiolysereaktion unterscheiden lassen. Acetoacetyl-CoA Thiolasen (EC 2.3.1.9), auch bekannt als Acetyl-CoA C-acetyltransferase, im Folgenden ACAT genannt, sind spezifisch für Acetoacetyl-CoA. Dagegen zeigen 3-Ketoacyl-CoA Thiolasen (EC 2.3.1.16) ein breites Kettenlängen-Spektrum für verschiedene β-Ketoacyl-CoA Substrate. Diese beiden Klassen lassen sich weiter unterteilen im Hinblick auf ihre subzelluläre Lokalisierung. Acetoacetyl-CoA Thiolasen befinden sich generell im Cytoplasma, wo sie im Mevalonat-Stoffwechselweg den ersten Reaktionsschritt vom Acetyl-CoA zum Acetoacetyl-CoA katalysieren (wobei es sich um die Rückreaktion der reversiblen thiolytischen Spaltung des Acetoacetyl-CoAs handelt), und in Mitochondrien, wo sie eine Funktion im Stoffwechsel der Ketonkörper besitzen. In Alkan-assimilierenden Pilzen wurden auch peroxisomale ACATs identifiziert (Kurihara et al., 1988, Kurihara et al., 1992, Yamagami et al., 2001). Im Gegensatz dazu sind 3-Ketoacyl-CoA Thiolasen generell in Mitochondrien und Peroxisomen lokalisiert und in die Fettsäure-β-oxidation involviert.

Zwei verschiedene Acetoacetyl-CoA Thiolase-Isoenzyme, cytoplasmatisch und mitochondrial, wurden für *Saccharomyces cerevisiae* (Kornblatt and Rudney, 1971b) beschrieben. Vermutlich wirkt die cytoplasmatische Acetoacetyl-CoA Thiolase anabolisch im Mevalonat-Stoffwechsel, wogegen die mitochondriale Acetoacetyl-CoA Thiolase die Poolgröße des Acetoacetyl-CoA, welches beim Fettsäurekatabolismus entsteht, und des Acetyl-CoA, welches in den Tricarbonsäure-Cyclus eintritt, reguliert. Die Größe der beiden Enzyme, geschätzt durch Gelfiltration, beträgt 140,000 Da für das cytoplasmatische Isoenzym und 65,000 Da für das mitochondriale Isoenzym. Beide Isoenzyme unterscheiden sich weiter in ihrer Fähigkeit, Dithiothreitol *in vitro* als Thioldonor zu verwenden (Kornblatt and Rudney 1971a). Es gibt keine immunologischen Daten oder Proteinsequenz-Informationen über den Verwandtschaftsgrad der beiden Isoenzyme.

Acetoacetyl-CoA Thiolase Gene wurden aus einer großen Zahl pro- und eukaryotischer Organismen isoliert. Acetoacetyl-CoA Thiolase übt in Bakterien eine Funktion in der Biosynthese von Poly-β-hydroxybuttersäure, einem Speichermolekül, aus. Bakterielle Gene, die für Acetoacetyl-CoA Thiolasen codieren, wurden z.B. aus *Zoogloea ramigera* (Peoples et al., 1987) und *Thiocystis violacea* (Liebergesell and Steinbüchel, 1993) isoliert. Gene, die für mitochondriale Acetoacetyl-CoA Thiolasen kodieren, wurden u.a. in menschlichen (Fukao et al., 1990) und Rattenzellen (Fukao et al., 1989) identifiziert. Die Hefe *Candida tropicalis* besitzt zwei ACAT-Isoenzyme (cytosolisch und peroxisomal), die von den gleichen 2 Genen kodiert werden (*CT-T1A* und *CT-T1B*; Kanayama et al., 1997). Das Produkt des Acetoacetyl-CoA Thiolase Gens aus der Hefe *Saccharomyces uvarum* (Dequin et al., 1988) ist vermutlich im Cytoplasma lokalisiert.

Ergosterol ist das Hauptprodukt des Mevalonat-Stoffwechselweges der Hefe *Saccharomyces cerevisiae* (vgl. Abb. 1 und Abb. 2). Ergosterol ist ein Bestandteil pilzlicher Zellmembranen und für das Pilzwachstum unerlässlich. Aus *S. cerevisiae* wurde das Gen *ERG10*, welches für ACAT codiert, als essentielles Gen beschrieben (vgl. Hiser et al., 1994). *ERG10* codiert für ein Polypeptid mit 398 Aminosäuren und einer errechneten molekularen Masse von 41681 Da. Über Gelfiltration konnte gezeigt werden, dass rekombinante ACAT der Hefe als Homotetramer mit einer Proteingröße von ca. 179 kDa vorliegt.

Die ACAT ist biochemisch sehr gut charakterisiert (vgl. Clinkenbeard et al., 1975 Dequin et al., 1988, Hiser et al., 1994). Die Kristallstruktur des Enzyms wurde aus dem Bakterium *Zoogloea ramigera* ermittelt (Modis et al., 1999).

Trotz dieser hervorragenden Charakterisierung der ACAT ist das Enzym jedoch niemals als Angriffspunkt für fungizide Wirkstoffe erkannt worden. Eine Nutzung dieses interessanten Enzyms beschränkt sich bisher auf die klinische Chemie, in der die ACAT zur Diagnose von Defekten am Ketonkörper-Metabolismus benutzt wird (Watanabe et al., 1998). Gleichwohl ist bekannt, dass sich ACAT aus verschiedenen Organismen (z.B. in der Rattenleber, vgl. Greenspan et al., 1989) durch organische Moleküle hemmen lässt.

Im Rahmen der vorliegenden Erfindung wurden die vollständige cDNA Sequenz und das entsprechende Gen (*acc1*) (vgl. SEQ ID NO. 1 und SEQ ID NO. 2) aus *Ustilago maydis* kodierend für ACAT isoliert [z.B. codierende Sequenz des *Ustilago maydis* Stammes 521, DSM Nr. 14603, Chromosom IX BAC Um31, Start 15266-15210 (Exon 1), 15073-14703 (Exon 2) und 14150-13356 (Exon 3), 15209-15074 (Intron 1) und 14702-14151 (Intron 2), entsprechend einer Länge von 408 Aminosäuren].

Der Brandpilz *Ustilago maydis,* ein Basidiomycet, befällt Maispflanzen. Die Krankheit kommt in allen Maisanbaugebieten vor, erreicht jedoch nur in trockenen Jahren eine größere Bedeutung. Typische Symptome sind die beulenartigen, faustgroßen Anschwellungen (Brandbeulen), die an allen oberirdischen Pflanzenteilen gebildet werden. Die Beulen sind zuerst von einer weiß-grauen, derben Haut überzogen. Beim Aufreißen der Haut wird eine schwarze, zunächst schmierige, später pulvrige Brandsporenmasse frei. Weitere phytopathogene Arten der Gattung *Ustilago* sind z.B. *U. nuda* (verursacht Gersten- und Weizenflugbrand), *U. nigra* (verursacht Gerstenschwarzbrand), *U. hordei* (verursacht Gerstenhartbrand) und *U. avenae* (verursacht Haferflugbrand).

Im Rahmen der vorliegenden Erfindung wurde mittels Knockout-Analysen im Basidiömyceten *Ustilago maydis* überraschend festgestellt, dass das Enzym ACAT wie im Ascomyceten *Saccharomyces cerevisiae* auch in diesem pflanzenpathogenen Pilz essentiell zum Überleben des Organismus ist (siehe auch Hiser et al., 1994). Daraus kann geschlossen werden, dass die ACAT nicht nur für einen spezifischen Pilz, hier *Saccharomyces cerevisiae,* sondern für Pilze im Allgemeinen eine besondere Rolle spielt. Die ACAT wurde damit erstmals als ein optimales Target für die Suche nach neuen, spezifischen Fungiziden, gerade in pflanzenpathogenen Pilzen, erkannt und damit die Möglichkeit gegeben, mit Hilfe dieses Targets gegebenenfalls völlig neue Leitstrukturen zu identifizieren, die die ACAT inhibieren und als Fungizide verwendet werden können.

Im Rahmen der vorliegenden Erfindung wurde weiter gefunden, dass die ACAT zum Identifizieren von Substanzen in geeigneten Testverfahren verwendet werden kann, die die Aktivität des Enzyms beeinflussen. Neben einer ACAT aus einem phytopathogenen Pilz, die durch ihre Aminosäuresequenz und die dafür kodierende Nukleinsäuresequenz charakterisiert wird, werden auch geeignete Testverfahren zum Identifizieren von Modulatoren des Enzyms zur Verfügung gestellt.

Im Rahmen der vorliegenden Erfindung wurde weiter gefunden, dass die ACAT auch tatsächlich durch Wirkstoffe inhibiert werden kann und ein mit diesen Wirkstoffen behandelter pilzlicher Organismus durch die Behandlung mit diesen Wirkstoffen geschädigt oder abgetötet werden kann, die Inhibitoren der ACAT also als Fungizide verwendet werden können. In der vorliegenden Erfindung wird beispielsweise gezeigt, dass die Hemmung der ACAT mit in einem Testsystem identifizierten Substanzen zum Absterben der behandelten Pilze in synthetischen Medien sowie auf der Pflanze führt.

Obwohl bereits bekannt war, dass das für die ACAT aus *Saccharomyces cerevisiae* kodierende Gen ein essentielles Gen ist, war bislang nicht bekannt, dass die ACAT in Pilzen ein Zielprotein (ein so genanntes "Target") fungizid wirksamer Substanzen sein kann. Damit wird in der vorliegenden Erfindung zum ersten Mal gezeigt, dass die ACAT ein für Pilze, insbesondere für phytopathogene Pilze lebenswichtiges Enzym darstellt und deshalb in besonderem Maße dazu geeignet ist, als Zielprotein für die Suche nach weiteren und verbesserten fungizid wirksamen Wirkstoffen verwendet zu werden.

Der Nachweis, dass ACAT auch für phytopathogene Pilze ein lebenswichtiges Enzym darstellt, wird bevorzugt durch die Herstellung von Deletionsmutanten erbracht. Dazu wendet man das in DE 101 33 928.3 beschriebene Verfahren an (siehe auch die Beispiele).

In der vorliegenden Erfindung wird weiterhin zum ersten Mal die ACAT aus *Ustilago maydis* beschrieben. Die beschriebene ACAT gehört zur oben beschriebenen Klasse von Thiolasen. Die für eine ACAT kodierende Sequenz aus einem Basidiomyceten war bislang noch nicht bekannt. In den Datenbanken sind die Gene für ACATs aus verschiedenen Pilzen (sämtlich Ascomyceten) hinterlegt, nämlich den nicht phytopathogenen *Saccharomyces cervisiae, Saccharomyces bayanus, Candida tropicalis, Yarrowia lipolytica, Schizosaccharomyces pombe* und dem phytopathogenen *Mycosphaerella graminicola.* Weiterhin befinden sich in den Datenbanken Fragmente der für ACAT kodierenden DNA aus dem pflanzenpathogenen Ascomyceten *Magnaporthe grisea,* den nicht pathogenen Ascomyceten *Neurospora crassa, Saccharomyces kluyveri* und *Zygosaccharomyces rouxii* sowie dem phytopathogenen Oomyceten *Phytophthora infestans* (siehe Tabelle 1).

**Tabelle 1:**

| Liste der Pilze, deren Protein- oder DNA-Sequenzen kodierend für die ACAT bekannt sind bzw. aus denen Teilsequenzen als kodierend für Teile der ACAT postuliert wurden. Die Herkunft der Sequenzinformation ist angegeben, ebenso die Ähnlichkeit und Identität der Sequenzen oder Sequenzfragmente zur ACAT aus *Ustilago maydis* (Aminosäureebene). | | | | |
|---|---|---|---|---|
| **Protein-Sequenzen** | | | | |
| **Organismus** | **Accession No.** | **Identität** (%) | **Ähnlichkeit** (%) | **Länge** (Aminosäuren) |
| *Candida* *tropicalis* | SWISS-PROT Q04677 | 55,4 | 64,1 | 402 |
| *Candida* *tropicalis* | SPTREMBL Q12598 | 55,4 | 64,2 | 402 |
| *Saccharomyces* *bayanus* | SWISS-PROT P10551 | 52,9 | 62 | 398 |
| *Saccharomyces* *cerevisiae* | SWISS-PROT P41338 | 54 | 61,5 | 398 |

| **Protein-Sequenzen** | | | | |
|---|---|---|---|---|
| **Organismus** | **Accession No.** | **Identität** (%) | **Ähnlichkeit** (%) | **Länge** (Aminosäuren) |
| *Mycosphaerella* *graminicola* | SPTREMBL Q9Y838 | 54,8 | 64,6 | 444 |
| *Schizosaccharo* *myces pombe* | SPTREMBL Q9UQW6 | 54,5 | 63,8 | 395 |
| *Yarrowia* *lipolytica* | SPTREMBL Q9C1T3 | 60,5 | 68,4 | 397 |
| | | | | |

| **DNA-Sequerizen** | | | | |
|---|---|---|---|---|
| **Organismus** | **Accession No.** | **Identität** (%) | **Ähnlichkeit** (%) | **Länge** (Basenpaare) |
| *Magnaporthe* *grisea* | EMBL AQ361828 | 58,7 | 66,8 | 723 |
| *Saccharomyces* *kluyveri* | EMBL AL405378 | 36,4 | 42 | 987 |
| *Zygosaccharom* *yces rouxii* | EMBL AL396273 | 48,3 | 56,9 | 943 |
| *Neurospora* *crassa* | EMBL AW713418 | 59,3 | 68,3 | 444 |
| *Phytophthora infestans* | EMBL BE777121 | 48,9 | 56,8 | 598 |

Mittels der bekannten Sequenz aus *Saccharomyces* sowie der erfindungsgemäßen Sequenz aus *Ustilago maydis* können die putativen, als ESTs bekannten Teilsequenzen als Sequenzen kodierend für ACAT bestätigt werden.

Auf Basis der im Rahmen dieser Erfindung durchgeführten Vergleiche (siehe auch Tabelle 1) lässt sich nunmehr sagen, dass die ACATs aus *Ustilago maydis* und aus weiteren Pilzen wie z.B. *Saccharomyces cerevisiae* oder *Yarrowia lipolytica* beträchtliche Homologien zueinander aufweisen, weshalb auch zur ACAT aus *Ustilago* *maydis* homologe Polypeptide, die von entsprechend homologen Nukleinsäuren kodiert werden, als molekulare Interaktionspartner (Targets) fungizider Wirkstoffe verwendet werden können. Dabei sind vor allem die homologen Nukleinsäuren bzw. Polypeptide aus pflanzenpathogenen Pilzen von Interesse. Besonders bevorzugt können dazu auch pflanzenpathogene Basidiomyceten verwendet werden. Aufgrund der hohen Homologie zwischen den ACATs können zum Identifizieren von Inhibitoren des Enzyms auch die ACATs aus humanpathogenen Pilzen (siehe Tabelle 1) bzw. die dafür kodierenden Nukleinsäuren verwendet werden, um Inhibitoren des Enzyms zu identifizieren. Inhibitoren der ACATs können in analoger Weise auch eine Wirkung gegen humanpathogene Pilze entfalten und als Antimycotica verwendet werden.

Die Verwendung von ACATs aus Pilzen, besonders aus Ascomyceten, Basidiomyceten und Oomyceten, ganz besonders aus phytopathogenen Pilzen bzw. phytopathogenen Basidiomyceten, und insbesondere aus *Ustilago maydis* zum Identifizieren von fungizid wirksamen Substanzen sind deshalb von der vorliegenden Erfindung voll umfasst.

Besonders bevorzugt handelt es sich bei den genannten homologen Polypeptiden um solche, die zur ACAT aus *Ustilago maydis* eine Identität von 60%, bevorzugt 80%, besonders bevorzugt 90% und besonders bevorzugt von 95% über eine Länge von wenigstens 20, vorzugsweise wenigstens 25, besonders bevorzugt wenigstens 30 und ganz besonders bevorzugt wenigstens 100 fortlaufenden Aminosäuren und ganz besonders bevorzugt über die Gesamtlänge aufweisen.

Solche zur ACAT aus *Ustilago maydis,* insbesondere zum Polypeptid gemäß SEQ ID NO. 4 homologen Polypeptide, die zum Identifizieren von fungiziden Wirkstoffen verwendet werden können, müssen nicht vollständige pilzliche ACATs darstellen, sondern können auch nur Fragmente davon sein, solange sie zumindest noch eine biologische Aktivität der vollständigen pilzlichen ACATs aufweisen. Polypeptide, die eine gleichartige biologische Aktivität wie eine ACAT mit einer Aminosäuresequenz gemäß SEQ ID NO. 4 ausüben, werden noch als erfindungsgemäß betrachtet. Dabei müssen die erfindungsgemäßen Polypeptide aus den oben genannten Gründen nicht von ACATs aus *Ustilago maydis* oder aus phytopathogenen Pilzen ableitbar sein. Als erfindungsgemäß werden vor allem auch solche Polypeptide betrachtet, die ACATs beispielsweise der folgenden Pilze entsprechen oder Fragmenten davon, die noch deren biologische Aktivität haben:

Plasmodiophoromycetes, Oomycetes, Chytridiomycetes, Zygomycetes, Ascomycetes, Basidiomycetes und Deuteromycetes, z.B.

Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder P. *brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder *P. graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia-Arten, wie beispielsweise *Tilletia caries;* Ustilago-Arten, wie beispielsweise *Ustilago nuda* oder *Ustilago avenae,* Pellicularia-Arten, wie beispielswiese *Pellicularia sasakii*, Pyricularia-Arten, wie beispielsweise *Pyricularia oryzae,* Fusarium-Arten, wie beispielsweise *Fusarium culmorum,* Botrytis-Arten, Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Alternaria-Arten, wie beispielsweise *Alternaria brassicae* oder Pseudocercosporella-Arten, wie beispielsweise *Pseudocercosporella herpotrichoides.*

Von besonderem Interesse sind z.B. auch *Magnaporthe grisea, Cochliobulus heterostrophus, Nectria haematococca* und Phytophtora species.

Fungizide Wirkstoffe, die mit Hilfe der erfindungsgemäßen ACATs gefunden werden, können auch mit ACATs aus humanpathogenen Pilzspezies interagieren, wobei die Interaktion mit den unterschiedlichen in diesen Pilzen vorkommenden ACATs nicht immer gleich stark sein muss.

Gegenstand der vorliegenden Erfindungen ist deshalb auch die Verwendung von Inhibitoren der ACAT zum Herstellen von Mitteln zur Behandlung von durch humanpathogene Pilze hervorgerufenen Erkrankungen.

Dabei sind die folgenden humanpathogenen Pilze von besonderem Interesse, die die genannten Krankheitsbilder hervorrufen können:

Dermatophyten, wie z.B. Trichophyton spec., Microsporum spec., *Epidermophyton floccosum* oder *Keratomyces ajelloi,* die z.B. Fußmykosen (Tinea pedis) hervorrufen,

Hefen, wie z.B. *Candida albicans,* der z.B. Soor-Ösophagitis und Dermatitis hervorruft, *Candida glabrata, Candida krusei* oder *Cryptococcus neoformans,* die z.B. pulmonale Cryptococcose und auch Torulose hervorrufen können,

Schimmelpilze, wie z.B. *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasaleMykosen) hervorrufen, *Rhinosporidium seeberi,* der z.B. chron. granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis,* der z.B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi,* der z.B. Keloid-Blastomykose und Lobo's Krankheit hervorruft, und *Sporothrix schenckii,* der z.B. Sporotrichose (granulomatöse Hautmykose) hervorruft.

Fungizide Wirkstoffe, die mit Hilfe der erfindungsgemäßen ACATs gefunden werden, können also auch mit ACATs aus anderen zahlreichen phytopathogenen Pilzspezies interagieren, wobei die Interaktion mit den unterschiedlichen in diesen Pilzen vorkommenden ACATs nicht immer gleich stark sein muss. Dies erklärt unter anderem die beobachtete Selektivität der an diesem Enzym wirksamen Substanzen.

Gegenstand der vorliegenden Erfindung sind deshalb Nukleinsäuren, die für vollständige ACATs aus pflanzenpathogenen Pilzen kodieren mit Ausnahme der in Tabelle 1 aufgeführten Sequenzfragmente aus *Blumeria graminis, Cladosporium fulvum* und *Mycosphaerella graminicola* mit den unter den angegebenen Accession Numbers hinterlegten Sequenzen.

Gegenstand der vorliegenden Erfindung sind insbesondere Nukleinsäuren, die für ACATs aus Basidiomyceten kodieren, bevorzugt aus pflanzenpathogenen Basidiomyceten und ganz besonders bevorzugt aus der Gattung Ustilago.

Gegenstand der vorliegenden Erfindung sind ganz besonders bevorzugt Nukleinsäuren, die für ACAT aus *Ustilago maydis* kodieren.

Gegenstand der vorliegenden Erfindung sind insbesondere bevorzugt Nukleinsäuren aus *Ustilago maydis* gemäß SEQ ID NO. 2 und SEQ ID NO. 3 die für ein Polypeptid gemäß SEQ ID NO. 4 oder aktive Fragmente davon kodieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugt handelt es sich bei den erfindungsgemäßen Nukleinsäuren um DNA-Fragmente, die der cDNA der erfindungsgemäßen Nukleinsäuren entsprechen.

Besonders bevorzugt umfassen die erfindungsgemäßen Nukleinsäuren eine Sequenz aus Pilzen ausgewählt aus
a) der Sequenz gemäß SEQ ID NO. 1, SEQ ID NO. 2 und SEQ ID NO. 3,
b) Sequenzen, die für ein Polypeptid kodieren, welches die Aminosäuresequenz gemäß SEQ ID NO. 4 umfasst,
c) Sequenzen, welche an die unter a) und/oder b) definierten Sequenzen bei einer Hybridisierungstemperatur von 35-52°C hybridisieren,
d) Sequenzen, welche eine zumindest eine 60%ige, bevorzugt eine 80%ige, besonders bevorzugt eine 90%ige und ganz besonders bevorzugt eine 95%ige Identität mit den unter a) und/oder b) definierten Sequenzen aufweisen,
e) Sequenzen, welche zu den unter a) und/oder b) definierten Sequenzen komplementär sind, und
f) Sequenzen, welche aufgrund der Degeneriertheit des genetischen Codes für dieselbe Aminosäuresequenz codieren wie die unter a) bis e) definierten Sequenzen.

Eine ganz besonders bevorzugte Ausführungsform der erfindungsgemäßen Nukleinsäuren stellt ein cDNA-Molekül mit der Sequenz gemäß SEQ ID NO. 1, SEQ ID NO. 2 oder SEQ ID NO. 3 kodierend für ACAT mit der SEQ ID NO. 4 aus *Ustilago maydis* dar.

Der Ausdruck "vollständige" ACAT wie er hierin verwendet wird, beschreibt die ACATs, die von der vollständigen kodierenden Region einer Transkriptionseinheit kodiert wird, beginnend mit dem ATG-Startcodon und umfassend alle informationstragenden Exonbereiche des im Herkunftsorganismus vorliegenden, für ACAT kodierenden Gens, sowie die für eine korrekte Termination der Transkription nötigen Signale.

Der Ausdruck "aktives Fragment" wie er hierin verwendet wird, beschreibt nicht mehr vollständige Nukleinsäuren kodierend für ACAT, die aber noch für Enzyme mit der biologischen Aktivität einer ACAT kodieren, und die eine für eine ACAT charakteristische Reaktion wie vorne beschrieben katalysieren können. Solche Fragmente sind kürzer als die oben beschriebenen vollständigen, für die ACAT kodierenden Nukleinsäuren. Dabei können sowohl an den 3'- und/oder 5'-Enden der Sequenz Nukleinsäuren entfernt worden sein, es können aber auch Teile der Sequenz deletiert, d. h. entfernt worden sein, die die biologische Aktivität der ACAT nicht entscheidend beeinträchtigen. Eine geringere oder gegebenenfalls auch eine erhöhte Aktivität, die aber noch die Charakterisierung bzw. Verwendung des resultierenden ACAT Fragments gestattet, wird dabei als ausreichend im Sinne des hier verwendeten Ausdrucks verstanden. Der Ausdruck "aktives Fragment" kann sich ebenso auf die Aminosäuresequenz der ACAT beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist.

Der Ausdruck "Gen", wie er hierin verwendet wird, ist die Bezeichnung für einen Abschnitt aus dem Genom einer Zelle, die für die Synthese einer Polypeptid-Kette verantwortlich ist.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin genannten oder ableitbaren Sequenzinformation beispielsweise DNA-Fragmente aus anderen phytopathogenen Pilzen als *Ustilago maydis* isoliert werden, welche für ACATs kodieren, welche dieselben oder ähnliche Eigenschaften einer der erfindungsgemäßen ACATs aufweisen.

Hybridisierungsbedingungen werden nach folgender Formel näherungsweise berechnet:

Die Schmelztemperatur Tm = 81,5°C + 16,6 log[c(Na⁺)] + 0,41(% G + C)) - 500/n (Lottspeich und Zorbas, 1998).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM Na⁺ (entspricht 0.1 x SSC) gewaschen. Wird eine RNA-Probe zur Hybridisierung verwendet, so ist der Schmelzpunkt um 10-15°C höher.

Bevorzugte Hybridisierungsbedingungen sind nachstehend angegeben:

Hybridisierungslösung: DIG Easy Hyb (Fa. Roche, ZZ) Hybridisierungstemperatur: 37°C bis 50°C, bevorzugt 42°C (DNA-DNA), 50°C (DNA-RNA).
1. Waschschritt: 2 x SSC, 0,1 % SDS 2 x5 min bei Raumtemperatur;
2. Waschschritt: 1 x SSC, 0,1 % SDS 2 x 15 min bei 50°C; bevorzugt 0,5 x SSC, 0,1 % SDS 2 x 15 min bei 65°C; besonders bevorzugt 0,2 x SSC, 2 x 15min bei 68°C.

Der Grad der Identität der Nukleinsäuren/Peptide wird vorzugsweise bestimmt mit Hilfe des Programms NCBI BLASTN Version 2.0.4. (Altschul et al., 1997).

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die T3-, T7- oder SP6-Promotoren für prokaryotische Zellen oder zellfreie Systeme, sowie ebespezifische Promotoren aus phytopathogenen Pilzen, z.B. der spezifische Promotor der erfindungsgemäß zu verwendenden ACAT.

Gegenstand der vorliegenden Erfindung sind ferner Vektoren, die eine erfindungsgemäße Nukleinsäure, eine erfindungsgemäße regulatorische Region oder ein erfindungsgemäßes DNA-Konstrukt enthalten.

Als Vektoren können alle in molekularbiologischen Laboratorien verwendete Phagen, Plasmide, Phagmide, Phasmide, Cosmide, YACs, BACs, künstliche Chromosomen oder Partikel, die für einen Partikelbeschuss geeignet sind, verwendet werden.

Ein bevorzugter Vektor ist pET21b (Novagen).

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, vorzugsweise *E. coli,* als auch eukaryotische Zellen, wie Zellen von *Saccharomyces cerevisiae, Pichia pastoris,* phytopathogenen Pilzen, Pflanzen, Froschoozyten und Zelllinien von Säugern.

Gegenstand der vorliegenden Erfindung sind weiterhin Polypeptide mit der biologischen Aktivität von ACATs, die von den erfindungsgemäßen Nukleinsäuren kodiert werden.

Bevorzugt umfassen die erfindungsgemäßen Polypeptide eine Aminosäuresequenz ausgewählt aus
a) der Sequenz gemäß SEQ ID NO. 4,
b) Sequenzen, welche eine zumindest 60%ige, bevorzugt eine 80%ige und besonders bevorzugt eine 90%ige Identität mit den unter a) definierten Sequenzen haben, und
c) Sequenzen, welche die gleiche biologische Aktivität aufweisen wie die unter a) definierten Sequenzen.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können.

Der Begriff "vollständige ACAT" wie er hierin verwendet wird, beschreibt eine ACAT, die kodiert wird von einer vollständigen kodierenden Region einer Transkriptionseinheit beginnend mit dem ATG-Startcodon und umfassend alle informationstragenden Exonbereiche des im Herkunftsorganismus vorliegenden, für die ACAT kodierenden Gens, sowie für eine korrekte Termination der Transkription nötigen Signale.

Die erfindungsgemäßen Polypeptide können im Vergleich zu den entsprechenden Regionen von natürlich vorkommenden ACATs Deletionen oder Aminosäuresubstitutionen aufweisen, solange sie zumindest noch eine biologische Aktivität der vollständigen ACATs ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| Ursprünglicher Rest | Substitution |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Gegenstand der vorliegenden Erfindung ist somit auch die Verwendung von Polypeptiden, welche zumindest eine biologische Aktivität einer ACAT ausüben und eine Aminosäuresequenz umfassen, die eine zumindest 60%ige, bevorzugt eine 80%ige Identität und besonders bevorzugt eine 95%ige Identität mit der Sequenz aus *Ustilago maydis* gemäß SEQ ID NO. 1 oder SEQ ID NO. 2 aufweisen.

Der Ausdruck "biologische Aktivität einer ACAT", wie er hierin verwendet wird, bedeutet die Fähigkeit, die Acetylierung von Acetyl-CoA zu katalysieren.

Die erfindungsgemäßen Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Die markierten Oligonukleotide können auch verwendet werden, um von mRNA z.B. aus phytopathogenen Pilzen hergestellte cDNA-Banken zu durchsuchen. Klone, an die die markierten Oligonukleotide hybridisieren, werden zur Isolierung der betreffenden DNA-Fragmente ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Zur Herstellung der erfindungsgemäßen Polypeptide, insbesondere des von der Nukleinsäuresequenz gemäß SEQ ID NO. 2 kodierten Polypeptids, können außerdem Wirtszellen, die erfindungsgemäßen Nukleinsäuren enthalten, unter geeigneten Bedingungen kultiviert werden. Die gewünschten Polypeptide können danach auf übliche Weise aus den Zellen oder dem Kulturmedium isoliert werden. Die Polypeptide können auch in *in vitro*-Systemen hergestellt werden.

Zur Herstellung der erfindungsgemäßen ACAT aus *Ustilago maydis* kann das Gen *accl* z.B. rekombinant in *Escherichia coli* exprimiert und aus *E. coli* Zellen eine Enzympräparation hergestellt werden.

Ein mögliches Reinigungsverfahren der ACAT basiert auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie oder Affinitätschromatographie.

Ein schnelles Verfahren zum Isolieren der erfindungsgemäßen Polypeptide, die von Wirtszellen unter Verwendung einer erfindungsgemäß zu verwendenden Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Sechs-His-Tag sein. Das Fusionsprotein kann dann an einer Nickel-NTA-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z.B. unter Anwendung von Gelfiltrations-, Reversphasenoder leicht hydrophoben Säulen), Gelfiltration, differentieller Präzipitation, lonenaustausch-Chromatographie und Affinitätschromatographie.

Die Ausdrücke "Isolierung" oder "Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Gegenstand der vorliegenden Erfindung sind auch Verfahren zum Auffinden von chemischen Verbindungen, die an die ACAT binden und deren Eigenschaften verändern. Aufgrund der wichtigen Funktion der ACAT stellen Modulatoren, die die Aktivität beeinflussen, neue fungizide Wirkstoffe dar. Modulatoren können Agonisten oder Antagonisten bzw. Inhibitoren oder Aktivatoren sein.

Gegenstand der vorliegenden Erfindung ist ebenfalls die Verwendung der erfindungsgemäßen Polypeptide in Verfahren zum Auffinden von chemischen Verbindungen, die an die ACAT binden und deren Eigenschaften verändern.

Auf Grund der Eigenschaft, als Inhibitoren der ACAT von Pilzen, insbesondere phytopathogener Pilze zu wirken, können mittels geeigneter Verfahren gefundene Verbindungen, die die ACAT inhibieren, auch als gegebenenfalls markierte Kompetitoren in Verfahren zum Auffinden von weiteren Inhibitoren der ACAT aus Pilzen verwendet werden, die nicht dieser Gruppe von Verbindungen angehören müssen.

Die Verwendung der erfindungsgemäßen Nukleinsäuren bzw. Polypeptide in einem erfindungsgemäßen Verfahren ermöglicht das Auffinden von Verbindungen, die an die erfindungsgemäßen Polypeptide binden. Diese können dann als Fungizide, z.B. bei Pflanzen oder als antimycotische Wirkstoffe bei Menschen und Tieren angewandt werden. Beispielsweise werden Wirtszellen, die die erfindungsgemäßen Nukleinsäuren enthalten und die entsprechenden Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Insbesondere ist ein Verfahren Gegenstand der vorliegenden Erfindung, dass zur Identifizierung von fungiziden Wirkstoffen geeignet ist, die an Polypeptide aus Pilzen mit der biologischen Aktivität einer ACAT binden, bevorzugt an ACAT aus phytopathogenen Pilzen, besonders bevorzugt an ACAT aus *Ustilago* und dazu homologe Polypeptide, die die vorstehend genannte Konsensussequenz aufweisen. Die Verfahren können jedoch auch mit einem zur ACAT homologen Polypeptid aus einer anderen als den hier genannten Spezies durchgeführt werden. Verfahren, die andere als die erfindungsgemäße ACAT verwenden, sind von der vorliegenden Erfindung umfasst.

Viele Testsysteme, die die Prüfung von Verbindungen und natürlichen Extrakten zum Ziel haben, sind auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semi-gereinigtes Protein. Sie sind geeignet für eine "erste" Prüfung, die in erster Linie darauf abzielt, einen möglichen Einfluss einer Substanz auf das Zielprotein zu detektieren.

Um Modulatoren aufzufinden, kann ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls die Aktivität der erfindungsgemäßen Polypeptide zu erhöhen oder zu hemmen, wird erkennbar an einer erhöhten oder verringerten Bindung des markierten Liganden oder an einer erhöhten oder verringerten Umsetzung des markierten Substrates. Moleküle, die gut binden und zu einer erhöhten Aktivität der erfindungsgemäßen Polypeptide führen, sind Agonisten. Moleküle, die gut binden, und die biologische Aktivität der erfindungsgemäßen Polypeptide hemmen, sind gute Antagonisten. Es kann sich dabei auch um Hemmstoffe der oben genannten fungiziden Stoffklasse handeln, jedoch können auch völlige neue Stoffklassen diese modulatorische Aktivität aufweisen.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf kolorimetrisch oder fluorimetrische Substrate, die in ein Produkt umgewandelt werden oder ein Reporter-Gen, das auf Veränderungen der Aktivität oder der Expression der erfindungsgemäßen Polypeptide anspricht oder andere bekannte Bindungstests. Umgekehrt ist es möglich Substrate einzusetzen, die in ein kolorimetrisch oder fluorimetrisch erfassbares Produkt umgewandelt werden. Dabei kann das Reportersystem aus mehreren gekoppelten Reaktionsschritten bestehen, an deren Ende ein entsprechend nachweisbares Produkt steht.

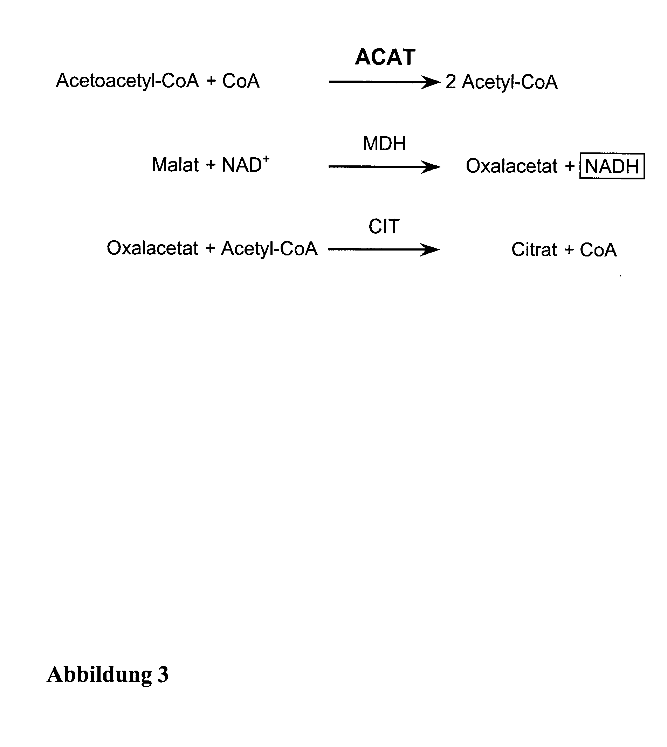

In einer bevorzugten Ausführungsform wird ein Testsystem verwendet, das neben der ACAT die Aktivität zweier weiterer Enzyme, nämlich Malat-Dehydrogenase (MDH) und Citrat-Synthase (CIT) benutzt, so dass die ACAT-Aktivität anhand er Zunahme der NADH-Konzentration, die photometrisch bestimmt wird, ermittelt werden kann. Während des Enzymtests laufen die in Abb. 3 gezeigten Reaktionen ab. Bei den gezeigten Reaktionen handelt es sich um reversible Reaktionen, deren Richtung sich durch die angebotenen Substrate beeinflussen lässt. Die im Enzymtest notwendige Richtung der Reaktion wird durch den Reaktionspfeil angegeben. Die MDH-Reaktion läuft normalerweise so ab, dass überwiegend Malat aus Oxalacetat gebildet wird. Durch die Kopplung mit der CIT-Reaktion wird das Oxalacetat allerdings verbraucht, Malat verstärkt durch MDH umgesetzt und somit das messbare NADH gebildet. Die NADH-Zunahme kann einfach fluorimetrisch (Anregung bei 340 nm/Emission bei 465 nm oder Anregung bei 360 nm/Emission bei 460 nm) gemessen werden.

Modulatoren, die in diesem gekoppelten Testsystem gefunden werden, werden in einem zweiten Testsystem, das nur die Enzyme MDH und CIT enthält, überprüft. Spezifische Modulatoren der ACAT werden dadurch erkannt, dass sie nur in dem Test mit ACAT, MDH und CIT zu einer erhöhten oder erniedrigten Aktivität führen, jedoch nicht in dem Test, der nur MDH und CIT enthält.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder kolorimetrisch, so dass man die Anzahl der Polypeptide, die an einen Liganden gebunden sind oder die eine Umsetzung mitgemacht haben, exakt bestimmen kann. Auf diese Weise lässt sich die Effektivität eines Agonisten oder Antagonisten ermessen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsäbhängige Testreihen überprüft werden. Kontrollansätze ohne Testsubstanzen können zur Bewertung der Effekte herangezogen werden.

Durch die anhand der vorliegenden Erfindung verfügbaren, die für ACAT kodierende Nukleinsäuren enthaltenden Wirtszellen, aber auch durch die anhand der vorliegenden Erfindung identifizierbaren entsprechenden, homologen ACATs aus anderen Spezies, wird die Entwicklung von Testsystemen, die auf Zellen basieren, zur Identifizierung von Substanzen ermöglicht, die die Aktivität der erfindungsgemäßen Polypeptide modulieren.

Eine andere Möglichkeit zur Identifizierung von Substanzen, die die Aktivtät der erfindungsgemäßen Polypeptide modulieren, ist auch der so genannte "Scintillation Proximity Assay" (SPA), siehe EP 015 473. Dieses Testsystem nutzt die Interaktion eines Polypeptids (z.B. ACAT aus *U. maydis)* mit einem radiomarkierten Liganden (z.B. ein kleines organisches Molekül oder ein zweites, radioaktiv markiertes Proteinmolekül). Das Polypeptid ist dabei an kleine Kügelchen ("Microspheres") oder Perlen ("Beads") gebunden, die mit szintillierenden Molekülen versehen sind. Im Verlauf des Abfalls der Radioaktivität wird die szintillierende Substanz im Kügelchen durch die subatomaren Partikel des radioaktiven Markers angeregt und ein detektierbares Photon emittiert. Die Testbedingungen werden so optimiert, dass nur jene vom Liganden ausgehenden Partikel zu einem Signal führen, die von einem an das erfindungsgemäße Polypeptid gebundenen Liganden ausgehen.

In einer möglichen Ausführungsform ist ACAT aus *U. maydis* an die Beads gebunden, entweder zusammen oder ohne interagierende bzw. bindende Testsubstanzen. Verwendet werden könnten dabei auch Fragmente des erfindungsgemäßen Polypeptids. Ein radioaktiv markierter Ligand könnte z.B. markiertes, nicht acetylierbares Acetyl-CoA-Analog sein. Bei einer Bindung eines bindenden Liganden an die immobilisierte ACAT, müsste dieser Ligand eine bestehende Interaktion zwischen der immobilisierten ACAT und dem markierten Liganden inhibieren oder aufheben, um selbst im Bereich der Kontaktfläche zu binden. Eine erfolgte Bindung an die immobilisierte ACAT kann dann anhand eines Lichtblitzes detektiert werden. Entsprechend wird ein bestehender Komplex zwischen einem immobilisierten und einem freien, markierten Liganden durch die Bindung einer Testsubstanz zerstört, was zu einem Abfall der detektierten Lichtblitzintensität führt. Das Testsystem entspricht dann einem komplementären Inhibitions-System.

Ein weiteres Beispiel für ein solches Testsystem ist das sogenannte "Two Hybrid System". Ein spezifisches Beispiel dafür ist die sogenannte "Interaction Trap", die Interaktions-Falle. Es handelt sich dabei um eine genetische Selektion von interagierenden Proteinen in Hefe (siehe z.B. Gyuris et al., 1993). Das Testsystem ist darauf ausgelegt, die Interaktion zweier Proteine zu detektieren und zu beschreiben, indem eine erfolgte Interaktion zu einem detektierbaren Signal führt.

Ein solches Testsystem kann auch an die Prüfung großer Zahlen von Testsubstanzen in einem gegebenen Zeitraum angepasst werden.

Das System beruht auf der Konstruktion zweier Vektoren, dem "Bait"- und dem "Prey"-Vektor. Ein für eine erfindungsgemäße ACAT oder Fragmente davon kodierendes Gen wird in den Bait-Vektor kloniert und dann als Fusionsprotein mit dem LexA-Protein, einem DNA-bindenden Protein, exprimiert. Ein zweites Gen, kodierend für ein Interaktionspartner von ACAT wird in den Prey-Vektor kloniert, wo es als Fusionsprotein mit dem B42-Prey-Protein exprimiert wird. Beide Vektoren liegen in einem *Saccharomyces cerevisiae-Wirt* vor, der Kopien von LexA-bindender DNA auf der 5'-Seite eines lacZ- oder HIS3-Reporter-Gens enthält. Findet eine Interaktion zwischen den beiden Fusions-Proteinen statt, kommt es zur Aktivierung der Transkription des Reporter-Gens. Führt die Anwesenheit einer Testsubstanz zur Inhibition oder zur Störung der Interaktion, können die beiden Fusions-Proteine nicht mehr interagieren und das Produkt des Reporter-Gens wird nicht mehr hergestellt.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt.

Der Ausdruck "Kompetitor" wie er hierin verwendet wird, bezieht sich auf die Eigenschaft der Verbindungen, mit anderen, gegebenenfalls noch zu identifizierenden Verbindungen um die Bindung an der ACAT zu kompetitieren und diese vom Enzym zu verdrängen bzw. von dieser verdrängt zu werden.

Der Ausdruck "Agonist", wie er hierin verwendet wird, bezieht sich auf ein Molekül, das die Aktivität der ACAT beschleunigt oder verstärkt.

Der Ausdruck "Antagonist", wie er hierin verwendet wird, bezieht sich auf ein Molekül, das die Aktivität der ACAT verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon.

Der Ausdruck "Fungizid" bzw. "fungizid", wie er hierin verwendet wird, stellt den Oberbegriff zu Substanzen zur Bekämpfung pflanzenschädigender Pilze und zu Substanzen zur Bekämpfung humanpathogener oder tierpathogener Pilze dar. Der Begriff umfasst damit auch Substanzen, die als Antimycotica verwendet werden können. In einer bevorzugten Bedeutung bezieht sich der Begriff auf Substanzen zur Bekämpfung pflanzenschädigender Pilze.

Vorzugsweise handelt es sich bei den Modulatoren um kleine organisch-chemische Verbindungen.

Die Bindung der Modulatoren an die ACAT kann die zellulären Vorgänge auf eine Weise verändern, die zum Absterben der damit behandelten Phytopathogenen Pilzen führt.

Gegenstand der vorliegenden Erfindung sind deshalb auch Modulatoren von ACATs aus phytopathogenen Pilzen, die mit Hilfe eines der in der vorliegenden Anmeldung beschriebenen Verfahrens zum Identifizieren von Modulatoren der ACAT gefunden werden.

Weiterhin umfasst die vorliegende Erfindung Verfahren zum Auffinden von chemischen Verbindungen, welche die Expression der erfindungsgemäßen Polypeptide verändern. Auch solche "Expressionsmodulatoren" können neue fungizide Wirkstoffe darstellen. Expressionsmodulatoren können kleine organisch-chemische Moleküle, Peptide oder Antikörper sein, die an die regulatorischen Regionen der für die erfindungsgemäßen Polypeptide kodierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die erfindungsgemäßen Polypeptide kodierenden Nukleinsäuren bindet, und dadurch deren Expression beeinflusst. Expressionsmodulatoren können auch Antisense-Moleküle sein.

Die vorliegende Erfindung bezieht sich ebenfalls auf die Verwendung von Modulatoren der erfindungsgemäßen Polypeptide oder von Expressionsmodulatoren als Fungizide.

Gegenstand der vorliegenden Erfindung sind ebenfalls Expressionsmodulatoren von ACATs, die mit Hilfe des vorstehend beschriebenen Verfahrens zum Auffinden von Expressionsmodulatoren gefunden werden.

Die erfindungsgemäßen Verfahren schließen Hochdurchsatz-Screening (high throughput screening; HTS) und Ultra-Hochdurchsatz-Screening (UHTS) ein. Dafür können sowohl Wirtszellen als auch zellfreie Präparationen verwendet werden, die die erfindungsgemäßen Nukleinsäuren und/oder die erfindungsgemäßen Polypeptide enthalten.

Gegenstand der Erfindung sind weiterhin Antikörper, die spezifisch an die erfindungsgemäßen Polypeptide oder Fragmente davon binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin lässt sich in an sich bekannter Weise eine immortalisierte Zelllinie erhalten, die monoklonale Antikörper produziert. Die Antikörper können gegebenenfalls mit einem Nachweisreagenz markiert sein. Bevorzugte Beispiele für ein solches Nachweisreagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften zeigen.

Die erfindungsgemäßen Nukleinsäuren können ebenfalls zur Herstellung transgener Organismen wie Bakterien, Pflanzen oder Pilze, bevorzugt zur Herstellung transgener Pflanzen und Pilze und besonders bevorzugt zur Herstellung transgener Pilze verwendet werden. Diese können z.B. in Testsystemen eingesetzt werden, die auf einer vom Wildtyp abweichenden Expression der erfindungsgemäßen Polypeptide oder Varianten davon basieren. Femer fallen hierunter sämtliche transgenen Pflanzen oder Pilze, bei denen durch die Modifikation anderer Gene als der vorstehend beschriebenen oder die Modifikation von Genkontrollsequenzen (z.B. Promotor) eine Veränderung der Expression der erfindungsgemäßen Polypeptide oder deren Varianten eintritt. Die transgenen Organismen sind auch zur (Über)Produktion des erfindungsgemäßen Polypeptids von Interesse, wobei z.B. Pilze (z.B. Hefe oder *Ustilago maydis),* die im Vergleich zur ihrer natürlichen Form das erfindungsgemäße Polypeptid vermehrt exprimieren, sich besonders zum Einsatz in Verfahren (gerade auch HTS-Verfahren) zum Identifizieren von Modulatoren des Polypeptids eignen.

Unter transgenen Organismen werden Organismen verstanden, in deren Genom mit Hilfe experimenteller Techniken heterologe (= fremde) Gene (= Transgene) stabil eingefügt und bevorzugt unter geeigneten Bedingungen zur Ausprägung gebracht werden.

Für die Herstellung transgener Pflanzen ist das am weitesten entwickelte Vektorsystem ein Plasmid aus dem Bakterium *Agrobacterium tumefaciens.* In der Natur infiziert *A. tumefaciens* Pflanzen und erzeugt Tumoren, die man als Wurzelhalsgallen bezeichnet. Diese Tumoren werden durch das Ti-Plasmid (tumor-inducing) von *A. tumefaciens* hervorgerufen. Das Ti-Plasmid baut ein Stück seiner DNA, das als T-DNA bezeichnet wird, in die chromosomale DNA der Wirtspflanze ein. Es wurde eine Möglichkeit entwickelt, die tumorinduzierenden Bereiche aus der DNA des Plasmids zu entfernen, aber seine Eigenschaft, genetisches Material in die Pflanzen einzubringen, beizubehalten. Dann kann mit Hilfe üblicher rekombinanter DNA-Techniken ein fremdes Gen, z.B. eine der erfindungsgemäßen Nukleinsäuren, in das Ti-Plasmid eingebaut werden. Das rekombinante Plasmid wird dann in *A. tumefaciens* wieder eingepflanzt, welches dann benutzt werden kann, um eine Pflanzenzellkultur zu infizieren. Man kann das Plasmid aber auch direkt in die Pflanzen einführen, wo es sich in die Chromosomen einbaut. Durch Regeneration solcher Zellen zu intakten Organismen bekommt man Pflanzen, die das Fremd-Gen enthalten und dieses auch exprimieren, d.h. das erwünschte Genprodukt produzieren.

*A. tumefaciens* infiziert dikotyle Pflanzen zwar leicht, aber für die Transformation von monokotylen Pflanzen, zu denen viele landwirtschaftlich wichtige Kulturpflanzen gehören, wie Mais, Weizen, Reis, ist es als Vektor von beschränktem Nützen, da es diese nicht ohne weiteres infiziert. Für die Transformation solcher Pflanzen stehen andere Techniken zur Verfügung, wie z.B. "DNA-Kanonen", das so genannte "particle gun" Verfahren. Bei diesem werden kleinste Kügelchen aus Titan oder Gold in Empfängerzellen oder -gewebe geschossen, entweder durch eine Gasentladung oder durch eine Pulverexplosion. Die Kügelchen sind mit DNA der interessierenden Gene beschichtet, wodurch diese in die Zellen gelangen, allmählich abgelöst und in das Genom der Wirtszellen eingebaut werden.

Nur wenige der Zellen, die dem fremden Erbmaterial ausgesetzt werden, sind in der Lage, dieses stabil in ihr eigenes Erbgut einzugliedern. In einem Gewebe, das für den Gentransfer verwendet wird, sind die nicht transgenen Zellen in der Überzahl. Während der Regeneration zur ganzen Pflanze ist es deshalb nötig, eine Selektion anzuwenden, welche die transgenen Zellen bevorteilt. In der Praxis verwendet man dafür Markergene, die in die Pflanzenzellen übertragen werden. Die Produkte dieser Gene inaktivieren einen Hemmstoff, etwa ein Antibiotikum oder Herbizid, und ermöglichen so den transgenen Zellen das Wachstum auf dem mit dem Hemmstoff versetzten Nährmedium. Weniger problematisch sind aber Gene, die für ein Enzym kodieren, das dann nachgewiesen werden kann. Dazu gehört auch das erfindungsgemäße Polypetid, dessen enzymatische Aktivität wie in Beispiel 2 beschrieben nachgewiesen werden kann.

Bei der Transformation mit *A*. *tumefaciens* können anstelle von Blattstückchen auch Protoplasten (isolierte Zellen ohne Zellwand, die in Kultur bei Anwesenheit bestimmter Chemikalien oder auch bei Elektroporation Fremd-DNA aufnehmen) benutzen. Man hält sie solange in Gewebekultur, bis sie eine neue Zellwand gebildet haben (z.B. bei Tabak etwa 2 Tage). Dann gibt man Agrobakterien zu und setzt die Gewebekultur fort. Eine einfache Methode zur transienten Transformation von Protoplasten mit einem DNA-Konstrukt ist die Inkubation in Gegenwart von Polyethylenglykol (PEG 4000).

Das Einbringen von DNA in Zellen ist auch durch Elektroporation möglich. Dies ist eine physikalische Methode zur Steigerung der DNA-Aufhahme in lebende Zellen. Durch elektrische Impulse lässt sich die Durchlässigkeit einer Biomembran kurzfristig erhöhen, ohne dass die Membran zerstört wird.

Das Einbringen von DNA ist ebenfalls durch Mikroinjektion möglich. Mit Hilfe von Glaskapillaren wird DNA in die Nähe des Zellkerns einer Zelle injiziert. Dieses ist bei pflanzlichen Zellen, die eine feste Zellwand und eine große Vakuole besitzen, allerdings schwierig.

Eine weitere Möglichkeit beruht auf der Nutzung von Ultraschall: Beschallt man Zellen mit Schallwellen oberhalb des menschlichen Hörbereichs (über 20 kHz), so wird ebenfalls vorübergehende Durchlässigkeit der Membranen boebachtet. Bei dieser Methode muss die Amplitude der Schallwellen sehr fein justiert werden, da die beschallten Zellen sonst platzen und zerstört werden.

Transgene Pilze können in dem Fachmann an sich bekannter Weise hergestellt werden (siehe auch Beispiele).

Gegenstand der Erfindung sind somit auch transgene Pflanzen oder Pilze, die zumindest eine der erfindungsgemäßen Nukleinsäuren enthalten, vorzugsweise transgene Pflanzen wie *Arabidopsis* species oder transgene Pilze wie Hefe species oder *Ustilago* species sowie deren transgene Nachkommen. Davon umfasst sind auch die Pflanzenteile, Protoplasten, Pflanzengewebe oder Pflanzenvermehrungsmaterialien der transgenen Pflanzen bzw. die Einzelzellen, Pilzgewebe, Fruchtkörper, Myzel und Sporen der transgenen Pilze, die die erfindungsgemäßen Nukleinsäuren enthalten. Vorzugsweise enthalten die transgenen Pflanzen oder Pilze die erfindungsgemäßen Polypeptide in einer vom Wildtyp abweichenden Form. Als erfindungsgemäß werden jedoch auch solche transgenen Pflanzen oder Pilze betrachtet, die natürlicherweise durch eine nur sehr geringe oder keine Expression des erfindungsgemäßen Polypeptids gekennzeichnet sind.

Gegenstand der vorliegenden Erfindung sind demnach ebenfalls transgene Pflanzen und Pilze, in denen Veränderungen an der für Polypeptide mit der Aktivität einer ACAT kodierenden Sequenz vorgenommen wurden und die dann mit Blick auf die Eignung zur Herstellung des erfindungsgemäßen Polypeptids und/oder eine durch Mutagenese erzielte Erhöhung oder Verminderung der biologischen Aktivität oder der Menge des in den Pflanzen oder Pilzen vorliegenden erfindungsgemäßen Polypeptids selektioniert wurden.

Der Ausdruck "Mutagenese" wie er hierin verwendet wird, bezeichnet eine Methode zur Erhöhung der spontanen Mutationsrate und damit zur Isolierung von Mutanten.

Dabei können Mutanten mit Hilfe von Mutagenen *in vivo* erzeugt werden, z.B. mit chemischen Verbindungen oder physikalischen Einflüssen, die geeignet sind, Mutationen auszulösen (z.B. Basenanaloga, UV-Strahlen etc.). Die gewünschten Mutanten können durch Selektion auf einen bestimmten Phänotyp hin erhalten werden. Die Position der Mutationen auf den Chromosomen kann relativ zu anderen, bekannten Mutationen durch Komplementations- und Rekombinationsanalysen bestimmt werden. Mutationen können auch gezielt in chromosomale oder extrachromosomale DNA eingebracht werden *(in vitro*-Mutagenese, site-directed Mutagense oder errorprone-PCR etc.).

Der Ausdruck "Mutante", wie er hierin verwendet wird, bezeichnet einen Organismus, der ein verändertes (mutiertes) Gen trägt. Eine Mutante ist definiert durch den Vergleich mit dem Wildtyp, der das unveränderte Gen trägt.

Die nachfolgenden Beispiele zeigen nun, dass die ACAT überraschenderweise ein essentielles Enzym in phytopathogenen Pilzen ist, und zeigen weiter, dass das Enzym ein geeignetes Zielprotein für die Identifizierung von Fungiziden ist, in Verfahren zum Identifizieren von fungizid wirksamen Verbindungen verwendet werden kann und dass die in entsprechenden Verfahren identifizierten Modulatoren der ACAT als Fungizide verwendet werden können.

Weiterhin wird beispielhaft die Gewinnung dieses Enzyms aus *Ustilago maydis* sowie aus *Saccharomyces cerevisiae* beschrieben und schließlich die Anwendbarkeit der vorliegenden Erfindung für die Suche nach fungizid wirksamen Verbindungen demonstriert.

Die folgenden Beispiele sind nicht beschränkt auf *Ustilago maydis.* Analoge Verfahren und Ergebnisse werden auch in Zusammenhang mit anderen phytopathogenen Pilzen erhalten.

### Beispiele

### Beispiel 1

### Funktionale Expression des ACAT Gens aus Saccharomyces cerevisiae in E. coli

Die über PCR amplifizierte kodierende Sequenz der ACAT (*ERG10*) aus *Saccharomyces cerevisiae* wird in den Expressionsvektor pET-21b (Fa. Novagen) in die *Nde*Iund *Xho*I-Schnittstellen kloniert, so dass ein C-terminales His-Tag angehängt wird. Mit diesem His-Tag-ACAT-Konstrukt (406 Aminosäuren) werden Zellen des Bakterienstammes NovaBlue BL21(DE) (Fa. Novagen) transformiert und als Glycerindauerkultur bei -80°C gelagert.

### Isolierung und Aufreinigung der ACAT aus Saccharomyces cerevisiae

Zellen aus der Glycerindauerkulturwerden auf eine frische LB Platte übertragen und auf Einzelkolonien ausgestrichen. Von einer Einzelkolonie wird eine Übernachtkultur (ca. 2 ml) angelegt und von dieser werden mit Hilfe von 20µl 250 ml LB-Medium (1% Tryptone, 0,5% Yeast Extract, 0,5% NaCl) mit Ampicillin (100 µg/ml) angeimpft und bei 37°C unter Schütteln inkubiert. Nach Erreichen einer OD₆₀₀ = 0,9 (nach 4-6h) wird die Expression des Polypeptids durch Zugabe von 1 mM IPTG (Endkonzentration) induziert und die Kultur unter Schütteln für 16 h bei 18°C weiter inkubiert. Die Zellen werden durch Zentrifugation [8000 rpm ≅ 10000g, 4°C, JA 14-Rotor, J2-21-Zentrifuge (Beckmann)] geerntet und bei -20°C eingefroren.

Das Pellet (1,5 g) wird in 15 ml Aufschlusspuffer (50 mM Tris/HCl, pH 8,0; 1 mM Glutathion) aufgenommen. Man gibt 30 mg Lysozym und 2,5 ml 1% Triton X-100 in Wasser zu und inkubiert für 30 min bei 28°C. Anschließend überführt man das Gefäß in ein Wasser/Ethanol/Eisbad und schließt die Zellen durch Ultraschall (Branson Sonifier 250, Benutzung des großen Sonifierkopfes, Output 50-55%, sechsmal je 30 Sekunden mit je 1 min Kühlpause) auf. Nach Zugabe von 0,8 ml 5 M Natriumchloridlösung (Endkonzentration 0,2 M) werden Zellbestandteile durch Zentrifugation für 30 min (J2-21, JA20 Rotor (Beckmann), 4°C, 10000g) abgetrennt. Der Überstand enthält die ACAT (Rohextrakt).

Die ACAT wird aus dem Überstand in Portionen von zweimal 10 ml durch Affinitätschromatographie gereinigt (2 mL Qiagen-Ni-NTA-Superflow-Säule, FPLC-Anlage der Fa. Pharmacia). Man verwendet dazu zwei Puffer:
- PufferA:: 50 mM Tris/HCl pH 8,0, 1 mM Glutathion
- PufferB:: 50 mM Tris/HCl pH 8,0, 1 mM Glutathion, 1 M Imidazol

Die Chromatographie erfolgt mit einem Gradientenprogramm:
1) 10 Säulenvolumina Puffer A
2) 10 Säulenvolumina Puffer A:B 995:5
   (Endkonzentration 5 mM Imidazol)
3) 10 Säulenvolumina Puffer A:B 90:10
   (Endkonzentration 100 mM Imidazol)
4) 5 Säulenvolumina Puffer B (Endkonzentration 1 M Imidazol)

Das Protein eluiert vollständig bei einer Konzentration von 100 mM Imidazol. Unter diesen Bedingungen ist die Säule überladen (Protein erscheint auch im Durchlauf).

Die Entsalzung des Proteins (10ml) erfolgt über vier PD10-Säulen bei 8°C mit 50 mM Tris/HCl pH 8,0, 1 mM Glutathion, 10%Glycerin.

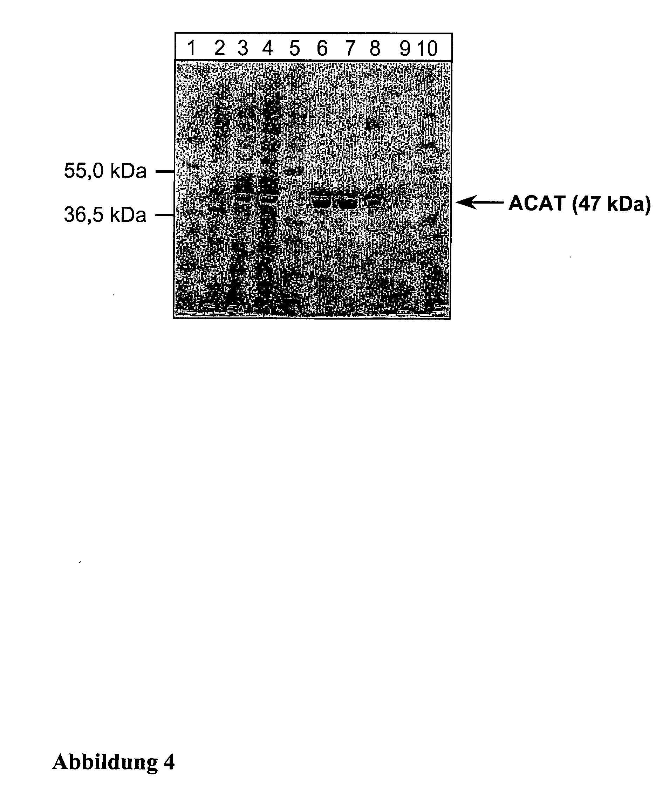

Man erhält ca. 15 mg an ACAT mit einer Reinheit von mehr als 99% (Coomassie-Färbung, vgl. Abb. 4).

Die ACAT verliert durch Einfrieren bei -80°C bzw. -20°C etwa 15% bzw. 20% ihrer Aktivität. Auf Eis ist eine Lagerung ohne größeren Aktivitätsverlust über mehrere Wochen möglich. Dieses Beispiel beschreibt die Stabilität der Enzym- und Substratlösung, die getrennt bei den verschiedenen Temperaturen aufbewahrt werden.

### Beispiel 2

### Funktionale Expression eines cDNA Klones des accl-Gens von Ustilago maydis in E. coli

Die über PCR amplifizierte kodierende Sequenz des *accl* Gens aus *U. maydis* wird in den Expressionsvektor pET-21b (Fa. Novagen) in die *NdeI-* und *Xho*I-Schnittstellen kloniert, so dass ein C-terminales His-Tag angehängt wird. Mit diesem His-Tag-ACAT-Konstrukt (408 + 2 Aminosäuren bedingt durch die Klonierung am C-Terminus + 6 Histidine = 416 Aminosäuren) werden Zellen des Bakterienstammes NovaBlue BL21(DE) (Fa. Novagen) transformiert und als Glycerindauerkultur bei -80°C gelagert.

### Isolierung und Aufreinigung der ACAT aus Ustilago maydis

Zellen aus der Glycerindauerkulturwerden auf eine frische LB Platte übertragen und auf Einzelkolonien ausgestrichen. Von einer Einzelkolonie wird eine Übernachtkultur (ca. 2 ml) angelegt und von dieser werden mit Hilfe von 20µl 250 ml LB-Medium (1% Tryptone, 0,5% Yeast Extract, 0,5% NaCl) mit Ampicillin (100 µg/ml) angeimpft und bei 37°C unter Schütteln inkubiert. Nach Erreichen einer OD₆₀₀ = 0,9 (nach 4-6h) wird die Expression des Polypeptids durch Zugabe von 1 mM IPTG (Endkonzentration) induziert und die Kultur unter Schütteln für 16 h bei 18°C weiter inkubiert. Die Zellen werden durch Zentrifugation [8000 rpm ≅ 10000g, 4°C, JA 14-Rotor, J2-21-Zentrifuge (Beckmann)] geerntet und bei -20°C eingefroren.

Das Pellet (1,5 g) wird in 15 ml Aufschlusspuffer (50 mM Tris/HCl, pH 8,0; 1 mM Glutathion) aufgenommen. Man gibt 30 mg Lysozym und 2,5 ml 1% Triton X-100 in Wasser zu und inkubiert für 30 min bei 28°C. Anschließend überführt man das Gefäß in ein Wasser/Ethanol/Eisbad und schließt die Zellen durch Ultraschall (Branson Sonifier 250, Benutzung des großen Sonifierkopfes, Output 50-55%, sechsmal je 30 Sekunden mit je 1 min Kühlpause) auf. Nach Zugabe von 0,8 ml 5 M Natriumchloridlösung (Endkonzentration 0,2 M) werden Zellbestandteile durch Zentrifugation für 30 min (J2-21, JA20 Rotor (Beckmann), 4°C, 10000g) abgetrennt. Der Überstand enthält die ACAT (Rohextrakt).

Die ACAT von Ustilago wird aus dem Überstand in Portionen durch Affinitätschromatographie gereinigt und in Analogie zu dem Enzym aus Hefe weiterbearbeitet und benutzt.

### Beispiel 3

### Enzymtest zum Auffinden von Modulatoren der ACAT

Je 5 µl der zu testenden Substanzen werden in einer 384-well Mikrotiterplatte in 5% (v/v) Dimethylsulfoxid vorgelegt. Zur Kontrolle wird eine Lösung von Iodacetamid, einem bekannten Inhibitor der ACAT, verwendet. Die Konzentration der Substanzen wird so bemessen, dass die Endkonzentration der Substanzen im durchgeführten Test zwischen 2 µM und 10 µM beträgt. Zu der Testsubstanzlösung werden 20 µl Enzym-Lösung (gekühlt bei 1-2°C, Zusammensetzung siehe unten) und 25 µl Substrat-Lösung (gekühlt bei 1-2°C, Zusammensetzung siehe unten) pipettiert. Es werden dabei ca. 100 - 500 ng Gesamtprotein eingesetzt, je nach spezifischer Aktivität der beteiligten Enzyme. Die Reaktion startet durch die Zugabe der Substrat-Lösung. Die Inkubation erfolgt bei Raumtemperatur für 25 min. Man misst die Abnahme der Fluoreszenz bei λ_{Ex} = 340 nm und λ_{Em} = 465 nm oder bei λ_{Ex} = 360 nm und λ_{Em} = 460 nm.

Die folgende Tabelle enthält die Angaben über alle im Assay verwendeten Substanzen (Stammlösung und Endkonzentrationen):

| | Stammlösung | Endkonzentration im Assay |
|---|---|---|
| Enzymlösung (20 µl): | | |
| 250 mM Tris/HCl pH 8,0 | 1 M | 100 mM |
| 25 mM Magnesiumchlorid | 1 M | 10 mM |
| 2,5 mM (L)-Malat | 100 mM | 1 mM |
| 6,25 mM Dithiothreitol | 100 mM | 2,5 mM |
| 2,5 mM NAD⁺⁽¹⁾ | 25 mM | 1 mM |
| 3,375 µM Coenzym A | 0,45 mM | 1,35 µM |
| 2,5 mM Glutathion | 100 mM | 1 mM |
| 12,5 % Glycerin | 50% | 5% |
| 250 µg/ml Bovines Serumalbumin | [20 mg/ml] | 100 µg/ml |
| 1,56 ng/µl Malatdehydrogenase | [10 µg/ml] | 31,25 ng/50 µl |
| 7,5 ng/µl Citratsynthase | [10 µg/ml] | 150 ng/50 µl |
| 0,34 ng/µl ACAT | [0,917 µg/ml] | 6,8 ng/50 µl |

| Substratlösung (25 µl): | | |
|---|---|---|
| 160 µM AcacCoA⁽²⁾ | 160 µM | 80 µM |

| Inhibitorlösung (5 µl): | | |
|---|---|---|
| z.B. Iodacetamid | 100 mM | 10 mM |
| Dimethylsulfoxid | 5% | 0,5% |

| | | |
|---|---|---|
| ⁽¹⁾ NAD: Nicotinamid-Adenin-Dinucleotid | | |
| ⁽²⁾ AcacCoA: Acetoacetyl-Coenzym A | | |

Es werden 20 µl Enzymlösung, 5 µl Inhibitorlösung bzw. Testsubstanzlösung und 25 µl Substratlösung verwendet.

Substanzen, die in dem beschriebenen Test eine Erhöhung oder Erniedrigung der Enzymaktivität bewirkt haben, werden in einem zweiten Test auf Modulation der MDH oder der CIT überprüft:

Je 5 µl der zu testenden Substanzen werden in einer 384-well Mikrotiterplatte in 5% (v/v) Dimethylsulfoxid vorgelegt. Die Konzentration der Substanzen wird so bemessen, dass die Endkonzentration der Substanzen im durchgeführten Test zwischen 2 µM und 10 µM beträgt. Zu der Testsubstanzlösung werden 20 µl Enzym-Lösung (gekühlt bei 1-2°C, Zusammensetzung siehe unten) und 25 µl Substrat-Lösung (gekühlt bei 1-2°C, Zusammensetzung siehe unten) pipettiert. Es werden dabei ca. 100 - 500 ng Gesamtprotein eingesetzt, je nach spezifischer Aktivität der beteiligten Enzyme. Die Reaktion startet durch die Zugabe der Substrat-Lösung. Die Inkubation erfolgt bei Raumtemperatur für 25 min. Man misst die Abnahme der Fluoreszenz bei λ_{Ex} = 340 nm und λ_{Em} = 465 nm oder bei λ_{Ex} = 360 nm und λ_{Em} = 460 nm.

Die folgende Tabelle enthält die Angaben über alle im Assay verwendeten Substanzen (Stammlösung und Endkonzentrationen):

| | Stammlösung | Endkonzentration im Assay |
|---|---|---|
| Enzymlösung (20 µl): | | |
| 250 mM Tris/HCl pH 8,0 | 1 M | 100 mM |
| 25 mM Magnesiumchlorid | IM | 10 mM |
| 2,5 mM (L)-Malat | 100 mM | 1 mM |
| 6,25 mM Dithiothreitol | 100 mM | 2,5 mM |
| 2,5 mM NAD⁺⁽¹⁾ | 25 mM | 1 mM |
| 3,375 µM Coenzym A | 0,45 mM | 1,35 µM |
| 2,5 mM Glutathion | 100 mM | 1 mM |
| 12,5 % Glycerin | 50% | 5% |
| 250µg/ml Bovines Serumalbumin | [20 mg/ml] | 100 µg/ml |
| 1,56 ng/µl Malatdehydrogenase | [10 µg/ml] | 31,25 ng/50 µl |
| 7,5 ng/µl Citratsynthase | [10 µg/ml] | 150 ng/50 µl |

| Substratlösung (25 µl): | | |
|---|---|---|
| 320 µM Acetyl-CoA | 320 µM | 160 µM |

| Testsubstanzlösung (5 µl): | | |
|---|---|---|
| Testsubstanz | | 2 µM bis 10 µM |
| Dimethylsulfoxid | 5% | 0,5% |

| | | |
|---|---|---|
| ⁽¹⁾NAD: Nicotinamid-Adenin-Dinucleotid | | |

Es werden 20 µl Enzymlösung, 5 µl Testsubstanzlösung und 25 µl Substratlösung verwendet.

### Beispiel 4

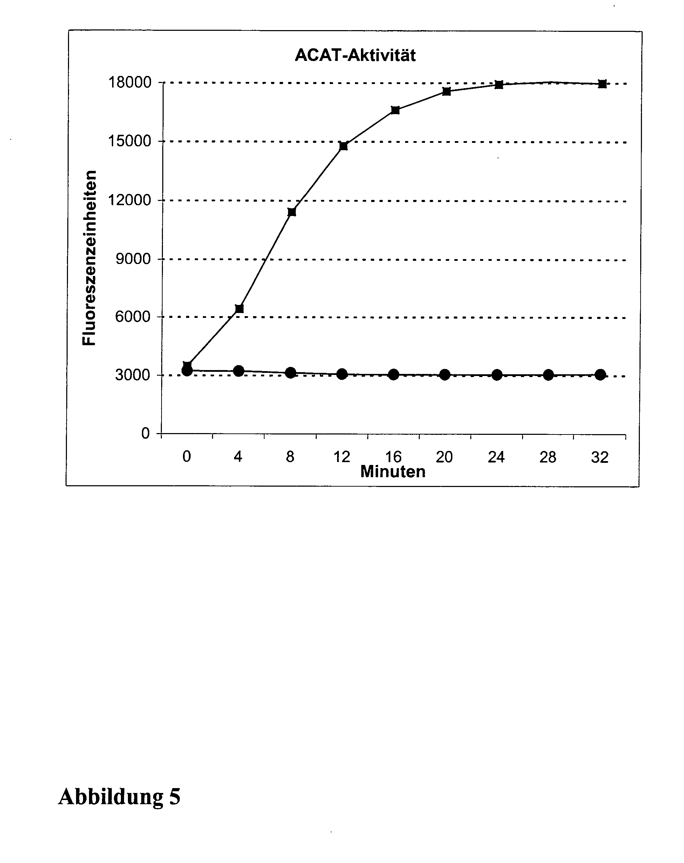

Die Enzymaktivität der ACAT in Abhängigkeit von der Zeit ist in Abb. 5 dargestellt (■). Der Kurvenverlauf in Anwesenheit eines unspezifischen Inhibitor ist zum Vergleich gezeigt (•).

### Beispiel 5

### Konstruktion der Hygromycin-Knockout-Kassette (hph-Kassette)

### Schritt 1

Das bakterielle Hygromycin-Resistenzgen (*hph*) wird aus dem Plasmid pCM54 (vgl. Tsukuda et al. 1988) durch PCR mit den Primern hph-Nco/Bam (SEQ ID NO. 7) und hph-Stop (SEQ ID NO. 8) amplifiziert (PCR-Protokoll nach Innis et al. 1990; Zyklen: a) 1 Zyklus von 10 min bei 94°C, b) 30 Zyklen von jeweils 1 min bei 94°C, 1 min bei 60°C, 3 min bei 72°C, c) 1 Zyklus von 10 min bei 72°C). Dadurch werden an beiden Flanken des *hph-Gens* unterschiedliche Restriktionsschnittstellen eingeführt: Nco I-Schnittstelle mit der Sequenz 5'-C↓CATGG-3' am ATG-Startcodon des *hph-Gens* und Not I-Schnittstelle mit der Sequenz 5'-GC↓GGCCGC-3' ein Nucleotid hinter dem STOP-Kodon des *hph-Gens.* Das PCR-Produkt wird anschließend mit den Enzymen Nco I und Not I (New England Biolabs, Bedingungen nach Angaben des Herstellers) restringiert.

### Schritt 2

In einem folgenden Schritt wird dieses PCR-Produkt, das das *hph-Gen* enthält, mit dem Agrobakterium NOS-Terminator verknüpft. Dazu wird das Plasmid potefSG (vgl. Spellig et al. 1996), welches zum Einen die Sequenz des Agrobakterium NOS-Terminators und zum Zweiten eine austauschbare Gensequenz für das sGFP-Gen enthält, mit den Enzymen Nco I und Not I (New England Biolabs, Bedingungen nach Angaben des Herstellers) restringiert. Durch die Restriktion wird das Fragment mit dem sGFP-Gen freigesetzt und gegen das PCR-Fragment mit dem *hph-*Gen ausgetauscht. Durch diese Klonierung wird das *hph-Gen* am 3'-Ende durch den Agrobakterium NOS-Terminator flankiert, um eine effektive Termination der *hph*-Transkription zu gewährleisten.

### Schritt 3

In einer zweiten PCR wird, ausgehend von diesem Plasmid, das *hph-Gen* zusammen mit dem NOS-Terminator amplifiziert. Man verwendet dazu die Primer hph-Nco/Bam (SEQ ID NO. 7) und 3-hph (SEQ ID NO. 9) (PCR-Protokoll nach Innis et al. 1990; Zyklen: a) 1 Zyklus von 10 min bei 94°C, b) 30 Zyklen von jeweils 1 min bei 94°C, 1 min bei 60°C, 3 min bei 72°C, c) 1 Zyklus von 10 min bei 72°C). Durch diese PCR werden am 5'-Ende die Schnittstellen für die Restriktionsenzyme BamH I (mit der Sequenz 5'-G↓GATCC-3') und Nco I und am 3'-Ende die Schnittstelle Sfi I-b gefolgt von einer Schnittstelle für das Enzym Sac I (Sequenz 5'-GAGCT↓C-3') generiert. Das PCR-Produkt wird anschließend direkt über TOPO-Klonierung (mit einem Kit der Fa. Invitrogen, Bedingungen nach Angaben des Herstellers) in das Plasmid pCR2.1 kloniert. Dadurch entsteht das Plasmid pBS-hph-Nos.

### Schritt 4

In einer dritten PCR wird, ausgehend von dem Plasmid pCM54 (siehe oben), durch die Primer 5-hsp (SEQ ID NO. 10) und hsp-Nco (SEQ ID NO. 11) ein 550 bp langes Fragment des *U. maydis hsp70*-Promotors amplifiziert (PCR-Protokoll nach Innis et al. 1990; Zyklen: a) 1 Zyklus von 10 min bei 94°C, b) 30 Zyklen von jeweils 1 min bei 94°C, 1 min bei 60°C, 3 min bei 72°C, c) 1 Zyklus von 10 min bei 72°C). Durch diese PCR wird am 5'-Ende der *hsp70*-Promotor-Sequenz eine Schnittstelle für das Restriktionsenzym Xho I (mit der Sequenz 5'-C↓TCGAG-3') und direkt darauf folgend die Schnittstelle Sfi I-a eingeführt. Am 3'-Ende wird eine Schnittstelle für das Enzym Nco I gefolgt von einer Schnittstelle für das Enzym HinDIII (mit der Sequenz 5'-A↓AGCTT-3') generiert. Das PCR-Produkt wird wiederum direkt über ein TOPO-Klonierung (mit einem Kit der Fa. Invitrogen, Bedingungen nach Angaben des Herstellers) in das Plasmid pCR2.1 kloniert. Dadurch entsteht das Plasmid pBS-hsp. Die korrekten Sequenzen der über PCR generierten Fragmente werden nach der Klonierung durch Sequenzierung verifiziert (ABI 377 Automated Sequencer, universal und reverse primer, Bedingungen nach Herstellerangaben unter Verwendung der Standard Universal- und Reverse-Primer).

### Schritt 5

In einem letzten Schritt wird i) das Plasmid pBS-hph-Nos mit den Enzymen Nco I und Sac I geschnitten (New England Biolabs, Bedingungen nach Angaben des Herstellers), wodurch das Fragment mit dem *hph*-Gen, der NOS-Terminator-Sequenz und der Schnittstelle Sfi I-b anfällt, ii) das Plasmid pBS-hsp mit den Enzymen Xho I und Nco I geschnitten (New England Biolabs, Bedingungen nach Angaben des Herstellers), wodurch das Fragment mit der *hsp70*-Promotor-Sequenz und der Schnittstelle Sfi I-a anfällt, iii) das käufliche Plasmid pBSKSII (Fa. Stratagene) mit Xho I und Sac I geschnitten (New England Biolabs, Bedingungen nach Angaben des Herstellers), und iv) die drei erhaltenen Fragmente gemeinsam ligiert (Ligase der Fa. Roche, Bedingungen nach Angaben des Herstellers), wodurch man das Plasmid pBS-hhn erhält. Aus diesem Plasmid kann die Hygromycin-Knockout-Kassette durch Restriktion mit Sfi I gewonnen und im erfindungsgemäßen Verfahren eingesetzt werden.

### Deletion des accl-Gens von Ustilago, welches für eine ACAT codiert

Durch PCR werden je ca. 750 bp lange Bereiche, die das ACAT Gen flankieren (im 3'-Bereich) bzw. in das zweite Exon hineinreichen (im 5'-Bereich), unter Verwendung der Primer mit den Sequenzen l*acc1*1 (SEQ ID NO. 12), l*acc1*2 (SEQ ID NO. 13), r*acc1*1 *(SEQ* ID NO. 14) und r*acc1*2 (SEQ ID NO. 15) generiert.

Die PCR wird unter folgenden Bedingungen durchgeführt: Nach einem ersten Denaturierungsschritt von 5 min bei 94°C folgen 30 PCR-Zyklen von je 1 min bei 94°C (Denaturierung), 1 min bei 62°C (Hybridisierung) und 1 min bei 72°C (Polymerase-Reaktion), worauf abschließend 7 min bei 72°C inkubiert wird (PCR-Protokoll nach Innis et al. 1990).

Durch die PCR Reaktion werden an den Enden der Fragmente, die an das *accl*-Gen grenzen bzw. darin enden, zwei verschiedene zu den Sfi I Schnittstellen der *hph*-Kassette kompatible Sfi I Schnittstellen eingeführt. Der Primer l*acc1*2 enthält dafür neben der Erkennungssequenz für den inneren linken flankierenden Bereich die Sequenz für die Sfi I-b-Schnittstelle sowie 3 weitere Nucleotide. Der Primer r*acc1*2 enthält dafür neben der Erkennungssequenz für den inneren rechten flankierenden Bereich die Sequenz für die Sfi I-a-Schnittstelle sowie 3 weitere Nucleotide.

Der Vektor pBS-hhn und die PCR generierten Fragmente werden entsprechend mit Sfi I restringiert, anschließend mit Phenol extrahiert, gelelektrophoretisch aufgereinigt und mit der *hph*-Kassette ligiert.

Die Restriktion der PCR-Fragmente erfolgt nach Ethanolfällung mit Sfi I (Fa. New England Biolabs, 20 Units, 2 h, Bedingungen der Enzymreaktion nach Herstellerangaben).

Die Reinigung der Fragmente erfolgt nach elektrophoretischer Auftrennung in Agarosegelen mit dem Gelelutions-Kit Jetsorb der Fa. Genomed nach Angaben des Herstellers.

Die Ligation der Flanken mit der *hph*-Kassette erfolgt in der Weise, dass man 0,2 µg jeder Flanke zusammen mit 0,2 µg der 2 kb langen *hph-*Kassette mit 2,5 Units Ligase (Fa. Roche, Bedingungen der Enzymreaktion nach Herstellerangaben) inkubiert.

Bedingt durch die nicht palindromischen, unterschiedlichen Überhänge der Sfi I Schnittstellen wird sowohl die Ligation von gleichen, als auch von verschiedenen Flanken verhindert. Eine Ligation über die stumpfen Enden der Fragmente wird ausgeschlossen, da die bei der PCR Reaktion eingesetzten Primer nicht phosphoryliert sind. In einer anschließenden PCR mit den entsprechenden Außenprimern (*laccl*1 und r*acc1*2) wird ein Gesamtprodukt von 3,5 kb amplifiziert.

Die PCR wird unter folgenden Bedingungen durchgeführt: Nach einem ersten Denaturierungsschritt von 4 min bei 94°C folgen 35 PCR-Zyklen von je 1 min bei 94°C (Denaturierung), 30 Sekunden bei 60°C (Hybridisierung) und 4,5 min bei 72°C (Polymerase-Reaktion), worauf abschließend 7 min bei 72°C inkubiert wird.

Das PCR Produkt von 3,5kb wird gefällt, in Wasser aufgenommen und direkt zur Transformation des *Ustilago maydis* Stammes FBD11 (*a1a2*/*b1b2*) eingesetzt (Beschreibung der Transformation siehe unten). Von dem Transformationsansatz werden 16 Kolonien isoliert und zur Infektion von Maispflanzen benutzt. Sporen der entstandenen Tumoren wurden einer Segregationsanalyse unterzogen. Wenn das *accl*-Gen auch in *Ustilago maydis* eine essentielle Funktion hat wird folgendes Muster in der Segregationsanalyse erwartet. Haploide Sporidien wachsen auf Medium ohne Hygromycin B. In Gegenwart von Hygromycin B ist kein Wachstum zu beobachten. In einem typischen Ergebnis ergab sich folgendes Bild. 11 Sporidien wuchsen in Abwesenheit von Hygomycin B jedoch nicht in Anwesenheit des Antibiotikums. Eine Kolonie wuchs unter beiden Bedingungen. In dieser Kolonie konnte durch PCR-Analyse ein Kopie des *acc1*-Gens und das Hygromycin-Resistenzgen nachgewiesen werden. In den anderen Stämmen wurde nur das *accl*-Gen nachgewiesen. Da keine Kandidaten gefunden wurden, denen das *accl*-Gen fehlt und die gleichzeitig resistent gegen Hygromycin B sind, ist das *accl*-Gen als essentielles Gen von *Ustilago maydis* eingestuft worden.

### Herstellung von Protoplasten von U. maydis und Transformation

50 ml *U. maydis*-Kultur in YEPS Medium werden bei 28°C bis zu einer Zelldichte von ca. 5 x 10⁷/ml (OD₆₀₀ 0.6 bis 1.0) angezogen. Dazu wird eine stationäre Vorkultur in drei Schritten 1:100, 1:300, 1:1000 verdünnt und für ca. 16 Stunden bei 28°C und 200 rpm in einem Kulturkolben mit Schikanen inkubiert. Nach Erreichen der gewünschten Zelldichte wird die Kultur für 7 min bei 2500 g abzentrifugiert. Das Zellpellet wird in 25 ml SCS-Puffer resuspendiert und erneut für 7 min bei 2500 g zentrifugiert. Das Pellet wird in 2 ml SCS-Puffer mit 2,5 mg/ml Novozym 234 (z.B. Fa. NovoBiolabs) resuspendiert. Die Protoplastierung erfolgt bei Raumtemperatur und wird mikroskopisch alle 5 min verfolgt. Die Protoplasten werden anschließend mit 10 ml SCS-Puffer gemischt und bei 1100 g für 10 min zentrifugiert. Der Überstand wird verworfen. Das Pellet wird vorsichtig in 10 ml SCS-Puffer resuspendiert, zentrifugiert und anschließend mit 10 ml STC-Puffer gewaschen und daraufhin in 500 µl kaltem STC-Puffer resuspendiert und auf Eis gehalten.

Zu maximal 7 µl linearer DNA (zwischen 3 µg und 5 µg) werden nacheinander 15 µg Heparin und 50 µl Protoplasten (in STC-Puffer) zugegeben und 10 min auf Eis gekühlt. Anschließend werden 500 µl PEG4000 [40 % (w/w) in STC-Puffer] zugesetzt, vorsichtig mit der Protoplastensuspension gemischt und für 15 min auf Eis inkubiert.

Zur Identifizierung von Transformanten wird der Transformationsansatz auf Agar-Platten ausplattiert (YEPS-Medium mit 1,5% Agar, 1 M Sorbitol und Antibiotikum; kurz vor dem Ausplattieren wird diese Agarschicht mit dem gleichen Volumen noch flüssigem Medium für Agar-Platten, das aber kein Antibiotikum enthält überschichtet). Das Ergebnis wird nach 3 bis 4 Tagen Inkubation bei 28°C bestimmt.
YEPS-Medium (vgl. Tsukuda et al. 1988)
   1 % Hefeextrakt, 2 % Bactopepton (Difco), 2 % Saccharose in Wasser.
SCS-Puffer: 20 mM Natriumcitrat, 1,0 M Sorbit in Wasser, pH 5,8.
STC-Puffer: 10 mM Tris/HCl (pH 7,5), 1,0 M Sorbit, 100 mM CaCl₂ in Wasser.
Medium für Agar-Platten zur Transformationskontrolle
   YEPS-Medium mit 1,5 % Agar, 1,0 M Sorbitol und Antibiotikum.
   Verwendete Konzentration unterschiedlicher Antibiotika im Agar-Medium zur Transformationskontrolle:
   Phleomycin 80 µg/ml; Carboxin 4 µg/ml; Hygromycin 400 µg/ml; ClonNAT 300 µg/ml.

### Erläuterungen zum Sequenzprotokoll

- SEQ ID NO. 1:: Genomische DNA-Sequenz für die ACAT *(acc1)* aus *Ustilago maydis* (Stamm 521, DSM Nr. 14603).
- SEQ ID NO. 2:: DNA-Sequenz des Gegenstranges der genomischen DNA-Sequenz entsprechend SEQ ID NO. 1, kodierend für die ACAT aus *Ustilago maydis* (Stamm 521, DSM Nr. 14603). Die Sequenz aus *Ustilago maydis* enthält zwei Introns.
- SEQ ID NO. 3:: cDNA Sequenz kodierend für die ACAT aus *Ustilago maydis.*
- SEQ ID NO. 4:: Aminosäuresequenz kodiert von der cDNA Sequenz gemäß SEQ ID NO. 3 kodierend für ACAT aus *Ustilago maydis.*
- SEQ ID NO. 5:: DNA-Sequenz kodierend für die ACAT aus *Saccharomyces cerevisiae.* Die Sequenz aus *S. cerevisiae* enthält keine Introns. Die kodierenden Bereiche sind erkennbar.
- SEQ ID NO. 6:: Aminosäuresequenz kodiert von dem Exon der Sequenz gemäß SEQ ID NO. 5 kodierend für ACAT aus *Saccharomyces cerevisiae.*
- SEQ ID NO. 7:: DNA-Sequenz des Primers hph-Nco/Bam.
- SEQ ID NO. 8:: DNA-Sequenz des Primers hph-STOP.
- SEQ ID NO. 9:: DNA-Sequenz des Primers 3-hph.
- SEQ ID NO. 10:: DNA-Sequenz des Primers 5-hsp.
- SEQ ID NO. 11:: DNA-Sequenz des Primers hsp-Nco.
- SEQ ID NO. 12:: DNA-Sequenz des Primers l*acc1*1
- SEQ ID NO. 13:: DNA-Sequenz des Primers l*acc1*2
- SEQ ID NO. 14:: DNA-Sequenz des Primers r*acc1*1
- SEQ ID NO. 15:: DNA-Sequenz des Primers r*acc1*2

### Erläuterungen zu den Abbildungen

- **Abbildung 1**:: Ergosterol-Biosynthese Teil 1: Vom Acetyl-CoA zum Squalen
- **Abbildung 2**:: Ergosterol-Biosynthese Teil 2: Vom Squalen zum Ergosterol

In Abb. 1 und Abb. 2 sind die einzelnen Zwischenstufen ausgehend vom Acetyl-CoA bis zum Ergosterol fett und schwarz angegeben. Die bekannten Enzyme sind neben dem Reaktionspfeil im Kasten angegeben, die zugehörigen Gene links vom Reaktionspfeil kursiv. Bekannte Targets (weißer Blockpfeil) mit den dazugehörigen Wirkstoffklassen (fett und unterstrichen) sind ebenfalls angegeben (vgl. Daum et al., 1998 und Wills et al., 2000).

### Abbildung 3: Enzymreaktionen im ACAT-Enzymtest

- ACAT:: Acetoacetyl-CoA Thiolase
- MDH:: Malat-Dehydrogenase
- CIT: Citrat-Synthase
- CoA: Coenzym A
- NAD⁺: Nicotinamid-adenin-dinukleotid (oxidierte Form)
- NADH: Nicotinamid-adenin-dinukleotid (reduzierte Form)

### Abbildung 4: SDS-Gelelektrophorese einzelner Fraktionen von der Isolierung der ACAT aus Saccharomyces cerevisiae

- Spur 1:: Größenmarker
- Spur 2:: Pellet (geerntete Zellen), ≈ 2 µg
- Spur 3:: Rohextrakt (nach Zellaufschluss), 10 µg
- Spur 4:: Durchlauf, 10 µg
- Spur 5:: Größenmarker
- Spur 6:: ACAT Fraktionen 6-8 nach PD-Säule, 5 µg
- Spur 7:: ACAT Fraktionen 6-8 nach FPLC, 5 µg
- Spur 8:: ACAT Fraktion 9 nach FPLC, ≈1 µg
- Spur 9:: Eluat mit Puffer B (1 M Imidazol)
- Spur 10:: Größenmarker

### Abbildung 5: Enzymatische Aktivtiät von ACAT.

- ―■―: ACAT-Aktivität in Abhängigkeit von der Zeit.
- ―•―: ACAT-Aktivität in Anwesenheit eines Inhibitors (10 mM Iodacetamid).

### Literatur

Altschul SF, Madden TL, Schaffer AA, Zhang JZ, Miller W, Lipman DJ (1997): Gapped BLAST und PSI-BLAST generation of protein database search programs. *Nucleic Acids Res.* **25,** 3389-3402.

Clinkenbeard KD, Sugiyama T, Lane MD (1975): Cytosolic acetoacetyl-CoA thiolase from chicken liver. *Meth. Enzymol.* **35,** 167-73.

Daum G, Lees ND, Bard M, Dickson R (1998): Biochemistry, cell biology and molecular biology of lipids *of Saccharomyces cerevisiae. Yeast* **14,** 1471-1510.

Dequin S, Gloeckler R, Herbert CJ, Boutelet F (1988): Cloning, sequencing and analysis of the yeast S. *uvarum ERG10* gene encoding acetoacetyl CoA thiolase. *Curr. Genet.* **13,** 471-478.

Fukao T, Kamijo K, Osumi T, Fujiki Y, Yamaguchi S, Orii T, Hashimoto T (1989): Molecular cloning and nucleotide sequence of cDNA encoding the entire precursor of rat mitochondrial acetoacetyl-CoA thiolase. *J. Biochem. (Tokyo)* **106,** 197-204.

Fukao T, Yamaguchi S, Kano M, Orii T, Fujiki Y, Osumi T, Hashimoto T (1990): Molecular cloning and sequence of the complementary DNA encoding human mitochondrial acetoacetyl-coenzyme A thiolase and study of the variant enzymes in cultured fibroblasts from patients with 3-ketothiolase deficiency. *J. Clin. Invest.* **86,** 2086-2092.

Greenspan MD, Yudkovitz JB, Chen JS, Hanf DP, Chan MN, Chiang PYC, Chabala JC, Alberts AW (1989): The Inhibition of Cytoplasmic Acetoacetyl-CoA Thiolase by a Triyne Carbonate (L-660,631). *Biochem. Biophys. Res. Commun.* **163,** 548-553.

Gyuris J, Golemis E, Chertkov H, Brent R (1993): Cdi1, a human G1 and S phase protein phosphatase that associates with Cdk2. *Cell* **75,** 791-803.

Hiser L, Basson ME, Rine J (1994): *ERG10* from *Saccharomyces cerevisiae* Encodes Acetoacetyl-CoA Thiolase. *J. Biol. Chem.* **269,** 31383-31389.

Innis M.A., Gelfand D.H., Sninsky J.J., White T.J., eds. (1990). PCR Protocols: A Guide to Methods and Applications. (Academic Press, San Diego, USA).

Kanayama N, Himeda Y, Atomi H, Ueda M, Tanaka A (1997): Expression of acetoacetyl-CoA thiolase isozyme genes of n-alkane-assimilating yeast, *Candida tropicalis:* isozymes in two intracellular compartments are derived from the same genes. *J. Biochem.* **122,** 616-21.

Kornblatt JA, Rudney H (1971a): Two forms of acetoacetyl coenzyme A thiolase in yeast. I. Separation and properties. *J. Biol. Chem.* **246,** 4417-4423.

Kornblatt JA, Rudney H (1971b): Two forms of acetoacetyl coenzyme A thiolase in yeast. II. Intracellular location and relationship to growth. *J. Biol. Chem.* **246,** 4424-4430.

Kurahashi Y, Sakawa S, Kinbara T, Tanaka K, Kagabu S (1997): Biological Activity of Carpropamid (KTU 3616): A New Fungicide for Rice Blast Disease. J. Pestic. Sci. 22, 108-112.

Kurihara T, Ueda M, Tanaka A (1988): Occurrence and possible roles of acetoacetyl-CoA thiolase and 3-ketoacyl-CoA thiolase in peroxisomes of an n-alkane-grown yeast, *Candida tropicalis. FEBS Lett.* **229,** 215-218.

Kurihara T, Ueda M, Kanayama N, Kondo J, Teranishi Y, Tanaka A (1992): Peroxisomal acetoacetyl-CoA thiolase of an n-alkane-utilizing yeast, *Candida tropicalis. Eur. J. Biochem.* **210,** 999-1005.

Liebergesell M, Steinbüchel A (1993): Cloning and molecular analysis of the poly(3-hydroxybutyric acid) biosynthetic genes of Thiocystis violacea. *Appl. Microbiol.* *Biotechnol.* **38,** 493-501.

Lottspeich F, Zorbas H (Hrsg.) (1998): Bioanalytik. Spektrum Akademischer Verlag, Heidelberg, Berlin.

Modis Y, Wierenga RK (1999): A biosynthetic thiolase in complex with a reaction intermediate: the crystal structure provides new insights into the catalytic mechanism. Structure Fold Des. 7, 1279-90.

Peoples OP, Masamune S, Walsh CT, Sinskey AJ (1987): Biosynthetic thiolase from Zoogloea ramigera. III. Isolation and characterization of the structural gene. *J*. *Biol. Chem.* **262,** 97-102.

Spellig T., Bottin A., Kahmann R. (1996). Green fluorescent protein (GFP) as a new vital marker in the phytopathogenic fungus Ustilago maydis, *Mol. Gen. Genet.* 252, 503-509.

Tsukuda T, Carleton S, Fotheringham S, Holloman WK (1988). Isolation and characterization of an autonomously replicating sequence from Ustilago maydis, *Mol. Cell. Biol.* **8,** 3703-3709.

Watanabe H, Yamaguchi S, Kimura M, Wakazono A, Song XQ, Fukao T, Orii T, Hashimoto T (1998): Practical Assay Method of Cytosolic Acetoacetyl-CoA Thiolase by Rapid Release of Cytosolic Enzymes from Cultured Lymphocytes Using Digitonin. *Tohoku J. Exp. Med.* **184,** 29-38.

Wills EA, Redinbo MR, Perfect JR, Del Poeta M (2000): New potential targets for antifungal development. *Emerging Therapeutic Targets 4, 1-32.*

Yamagami S, Toshiya I, Yuji N, Akinori O, Masamichi T (2001): Isolation and Characterization of Acetoacetyl-CoA Thiolase Gene Essential *for n*-Decane Assimilation in Yeast *Yarrowia lipolytica. Biochem. Biophys. Res. Comm.* **282,** 832-838.

## Patentansprüche

1. Nukleinsäure, kodierend für ein Polypeptid mit der Aktivität einer ACAT aus pflanzenpathogenen Pilzen.

2. Nukleinsäure gemäß Anspruch 1, **dadurch gekennzeichnet, dass** sie für ACAT aus Basidiomyceten kodiert.

3. Nukleinsäure gemäß Anspruch 1 oder 2, **dadurch gekennzeichnet, dass** sie für ACAT aus *Ustilago* kodiert.

4. Nukleinsäuren gemäß Anspruch 1, 2 oder 3, **dadurch gekennzeichnet, dass** es sich um einzelsträngige oder doppelsträngige DNA oder RNA handelt.

5. Nukleinsäuren gemäß einem der Ansprüche 1 bis 4, **dadurch gekennzeichnet, dass** es sich um Fragmente genomischer DNA oder um cDNA handelt.

6. Nukleinsäuren gemäß einem der Ansprüche 1 bis 5, umfassend eine Sequenz ausgewählt aus
a) einer Sequenz gemäß SEQ ID NO. 1, SEQ ID NO. 2 und SEQ ID NO. 3,
b) Sequenzen, die für ein Polypeptid codieren, welches die Aminosäuresequenz gemäß SEQ ID NO. 4 umfasst,
c) Sequenzen, welche an die unter a) und/oder b) definierten Sequenzen bei einer Hybridisierungstemperatur von 35-52°C hybridisieren,
d) Sequenzen, welche eine zumindest eine 60%ige, bevorzugt eine 80%ige, besonders bevorzugt eine 90%ige und ganz besonders bevorzugt eine 95%ige Identität mit den unter a) und/oder b) definierten Sequenzen aufweisen,
e) Sequenzen, welche zu den unter a) und/oder b) definierten Sequenzen komplementär sind, und
f) Sequenzen, welche aufgrund der Degeneriertheit des genetischen Codes für dieselbe Aminosäuresequenz codieren wie die unter a) bis e) definierten Sequenzen.

7. DNA-Konstrukt umfassend eine Nukleinsäure gemäß einem der Ansprüche 1 bis 6 und einen heterologen Promotor.

8. Vektor umfassend eine Nukleinsäure gemäß einem der Ansprüche 1 bis 6, oder ein DNA-Konstrukt gemäß Anspruch 7.

9. Vektor gemäß Anspruch 8, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

10. Wirtszelle enthaltend eine Nukleinsäure gemäß einem der Ansprüche 1 bis 6, ein DNA-Konstrukt gemäß Anspruch 7 oder einen Vektor gemäß Anspruch 8 oder 9.

11. Wirtszelle gemäß Anspruch 10, **dadurch gekennzeichnet, dass** es sich um eine prokaryotische Zelle handelt.

12. Wirtszelle gemäß Anspruch 10, **dadurch gekennzeichnet, dass** es sich um eine eukaryotische Zelle handelt.

13. Polypeptid mit der biologischen Aktivität einer ACAT, welches von einer Nukleinsäure gemäß einem der Ansprüche 1 bis 6 codiert wird.

14. Polypeptid mit der biologischen Aktivität einer ACAT, ausgewählt aus
a) der Sequenz gemäß SEQ ID NO. 4,
b) Sequenzen, welche eine zumindest 60%ige, bevorzugt eine 80%ige und besonders bevorzugt eine 90%ige Identität mit den unter a) definierten Sequenzen haben, und
c) Sequenzen, welche die gleiche biologische Aktivität aufweisen wie die unter a) definierten Sequenzen.

15. Antikörper, welcher spezifisch an ein Polypeptid gemäß Anspruch 13 oder 14 bindet.

16. Verfahren zum Herstellen einer Nukleinsäure gemäß einem der Ansprüche 1 bis 6, umfassend die folgenden Schritte:
(a) Vollständige chemische Synthese auf an sich bekannte Weise oder
(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Pilzzellen hergestellt wurde, Selektieren von positiven Klonen und Isolieren der hybridisierenden DNA aus positiven Klonen oder
(c) chemische Synthese von Oligonukleotiden und Amplifizierung der Ziel-DNA mittels PCR.

17. Verfahren zum Herstellen eines Polypeptids gemäß Anspruch 13 oder 14, umfassend
(a) das Kultivieren einer Wirtszelle gemäß einem der Ansprüche 10 bis 12 unter Bedingungen, die die Expression der Nukleinsäure gemäß einem der Ansprüche 1 bis 6 gewährleisten, oder
(b) das Exprimieren einer Nukleinsäure gemäß einem der Ansprüche 1 bis 6 in einem *in vitro*-System, und
(c) die Gewinnung des Polypeptids aus der Zelle, dem Kulturmedium oder dem *in vitro*-System.

18. Verfahren zum Auffinden einer chemischen Verbindung, die an ein Polypeptid gemäß Anspruch 13 oder 14 bindet und/oder die Aktivität dieses Polypeptids moduliert, umfassend die folgenden Schritte:
(a) Inkontaktbringen einer Wirtszelle gemäß einem der Ansprüche 10 bis 12 oder eines Polypeptids gemäß Anspruch 13 oder 14 mit einer chemischen Verbindung oder einem Gemisch von chemischen Verbindungen unter Bedingungen, die die Interaktion einer chemischen Verbindung mit dem Polypeptid erlauben, und
(b) Bestimmen der chemischen Verbindung, die spezifisch an das Polypeptid bindet und/oder die Aktivität dieses Polypeptids moduliert.

19. Verfahren zum Auffinden einer Verbindung, welche die Expression von Polypeptiden gemäß Anspruch 13 oder 14 verändert, umfassend die folgenden Schritte:
(a) Inkontaktbringen einer Wirtszelle gemäß einem der Ansprüche 10 bis 12 mit einer chemischen Verbindung oder einem Gemisch von chemischen Verbindungen,
(b) Bestimmen der Polypeptidkonzentration, und
(c) Bestimmen der Verbindung, welche die Expression des Polypeptids spezifisch beeinflusst.

20. Verwendung von ACAT aus Pilzen, von dafür kodierenden Nukleinsäuren, DNA-Konstrukten oder Wirtszellen enthaltend diese Nukleinsäuren zum Auffinden von fungiziden Wirkstoffen.

21. Verwendung eines Modulators eines Polypeptids mit der biologischen Aktivität einer ACAT als Fungizid.

22. Verwendung eines Modulators eines Polypeptids mit der biologischen Aktivität einer ACAT zum Herstellen eines Mittels zur Bekämpfung von schädlichen Pilzen.

23. Modulatoren, welche durch ein Verfahren gemäß Anspruch 18 oder 19 identifiziert werden.

24. Fungizid wirksame Substanzen, die mittels eines Verfahrens gemäß Anspruch 18 oder 19 gefunden werden.

25. Verwendung einer Nukleinsäure gemäß einem der Ansprüche 1 bis 6, eines DNA-Konstrukts gemäß Anspruch 7 oder eines Vektors gemäß Ansprüchen 8 oder 9 zum Herstellen von transgenen Pflanzen und Pilzen.

26. Transgene Pflanzen, Pflanzenteile, Protoplasten, Pflanzengewebe oder Pflanzenvermehrungsmaterialien, **dadurch gekennzeichnet, dass** nach Einbringen einer Nukleinsäure gemäß einem der Ansprüche 1 bis 6, eines DNA-Konstrukts gemäß Anspruch 7 oder eines Vektors gemäß Ansprüchen 8 oder 9 die intrazelluläre Konzentration eines Polypeptids gemäß Ansprüchen 13 oder 14 im Vergleich zu den entsprechenden Wildtyp-Zellen erhöht oder vermindert ist.

27. Transgene Pilze, Pilzzellen, Pilzgewebe, Fruchtkörper, Mycele und Sporen, **dadurch gekennzeichnet, dass** nach Einbringen einer Nukleinsäure gemäß einem der Ansprüche 1 bis 6, eines DNA-Konstrukts gemäß Anspruch 7 oder eines Vektors gemäß Ansprüchen 8 oder 9 die intrazelluläre Konzentration eines Polypeptids gemäß Ansprüchen 13 oder 14 im Vergleich zu den entsprechenden Wildtyp-Zellen erhöht oder vermindert ist.

28. Pflanzen, Pflanzenteile, Protoplasten, Pflanzengewebe oder Pflanzenvermehrungsmaterialien, **dadurch gekennzeichnet, dass** sie ein Polypeptid gemäß Ansprüchen 13 oder 14 enthalten, dessen biologische Aktivität oder Expressionsmuster im Vergleich zu den entsprechenden endogenen Polypeptiden verändert ist.

29. Verfahren zum Herstellen von Pflanzen, Pflanzenteilen, Protoplasten, Pflanzengeweben oder Pflanzenvermehrungsmaterialien gemäß Anspruch 28, **dadurch gekennzeichnet, dass** man eine Nukleinsäure gemäß einem der Ansprüche 1 bis 6 durch endogene Mutagenese verändert.

30. Pilze, Pilzzellen, Pilzgewebe, Fruchtkörper, Mycele und Sporen, **dadurch gekennzeichnet, dass** sie ein Polypeptid gemäß Ansprüchen 13 oder 14 enthalten, dessen biologische Aktivität oder Expressionsmuster im Vergleich zu den entsprechenden endogenen Polypeptiden verändert ist.

31. Verfahren zum Herstellen von Pilzen, Pilzzellen, Pilzgewebe, Mycelen und Sporen gemäß Anspruch 30, **dadurch gekennzeichnet, dass** man eine Nukleinsäure gemäß einem der Ansprüche 1 bis 6 durch endogene Mutagenese verändert.
